# FASCICULE DE BREVET EUROPEEN

(11) **EP 2 046 791 B1**
(45) Date de publication et mention de la délivrance du brevet: **24.03.2010**
(21) Numéro de dépôt: 07803782.7
(22) Date de dépôt: 26.06.2007
(51) Int. Cl.: C07D 471/08, A61K 31/46, A61P 3/04, A61P 3/10, A61P 9/10, A61P 19/10, A61P 25/28

(54) **DÉRIVÉS D'UREES DE TROPANE, LEUR PRÉPARATION ET LEUR APPLICATION EN THÉRAPEUTIQUE**
HARNSTOFFDERIVATE VON TROPAN, IHRE HERSTELLUNG UND IHRE THERAPEUTISCHE ANWENDUNG
UREA DERIVATIVES OF TROPANE, THEIR PREPARATION AND THEIR THERAPEUTIC APPLICATION

(30) Priorité: 27.06.2006 FR 0605785; 25.01.2007 FR 0700507
(43) Date de publication de la demande: 15.04.2009
(73) Titulaire: Sanofi-Aventis, 75013 Paris (FR)
(72) Inventeur: ALETRU, Michel C/o Sanofi-Aventis Département Brevets Tri E2/300, FR-92160 Antony (FR); BRAUN, Alain C/o Sanofi-Aventis Département Brevets Tri E2/300, FR-92160 Antony (FR); NAMANE, Claudie C/o Sanofi-Aventis Département Brevets Tri E2/300, FR-92160 Antony (FR); VENIER, Olivier C/o Sanofi-Aventis Département Brevets Tri E2/300, FR-92160 Antony (FR); PHILIPPO, Christophe C/o Sanofi-Aventis Département Brevets Tri E2/300, FR-92160 Antony (FR); MOUGENOT, Patrick C/o Sanofi-Aventis Département Brevets Tri E2/300, FR-92160 Antony (FR); NICOLAI, Eric C/o Sanofi-Aventis Département Brevets Tri E2/300, FR-92160 Antony (FR); GUSSREGEN, Stefan C/o Sanofi-Aventis Deutschland GMBH, 65926 Frankfurt (DE)
(74) Mandataire: Romanowski, Caroline
(86) Numéro de dépôt international: PCT/FR2007/001070
(87) Numéro de publication internationale: WO 2008/000951

(56) Documents cités:
- WO-A-2004/089896
- WO-A2-03/106456

## Description

La présente invention se rapporte à des dérivés d'urée de tropane, à leur préparation et à leur application en thérapeutique. Les présents composés modulent l'activité de la 11β-hydroxystéroïde déhydrogénase type 1 (11βHSD1) et sont utiles pour le traitement des pathologies dans lesquelles une telle modulation est bénéfique, comme dans le cas du syndrome métabolique ou du diabète de type 2 non insulino dépendant.

La 11β-hydroxystéroïde déhydrogénase type 1 (11βHSD1) catalyse localement la conversion de glucocorticoïdes inactifs (cortisone chez l'homme) en glucocorticoïdes actifs (cortisol chez l'homme) dans différents tissus et organes, principalement le foie et le tissu adipeux, mais aussi dans les muscles, les os, le pancréas, l'endothélium, le tissu oculaire et dans certaines parties du système nerveux central. La 11βHSD1 agit comme un régulateur de l'action des glucocorticoïdes dans les tissus et organes où elle est exprimée (Tomlinson et al., Endocrine Reviews 25(5), 831-866 (2004), Davani et al., J. Biol. Chem. 275, 34841 (2000) ; Moisan et al., Endocrinology, 127, 1450 (1990)).

Les pathologies les plus importantes dans lesquelles interviennent les glucocorticoïdes et l'inhibition de la 11βHSD1 sont indiquées ci-après.

### A. Obésité, diabète de type 2 et syndrome métabolique

Le rôle de la 11βHSD1 dans l'obésité, le diabète de type 2 et le syndrome métabolique (aussi connu sous le nom de syndrome X ou syndrome de résistance à l'insuline) où les symptômes incluent l'obésité viscérale, l'intolérance au glucose, la résistance à l'insuline, l'hypertension, le diabète de type 2 et l'hyperlipidémie (Reaven Ann. Rev. Med 44, 121 (1993)) est décrit dans de nombreuses publications. Chez l'homme, le traitement par la carbenoxolone (un inhibiteur non spécifique de la 11βHSD1) améliore la sensibilité à l'insuline chez des patients volontaires minces et chez des diabétiques de type 2 (Andrews et al., J. Clin. Endocrinol. Metab. 88, 285 (2003)). De plus les souris, dont le gène de la 11βHSD1 a été éteint, sont résistantes à l'hyperglycémie induite par le stress et l'obésité, montrent une atténuation de l'induction d'enzymes hépatiques de la néoglucogenèse (PEPCK et G6P) et présentent une augmentation de la sensibilité à l'insuline dans le tissu adipeux (Kotelevstev et al., Proc. Nat Acad. Sci. 94, 14924 (1997) ; Morton et al., J. Biol. Chem. 276, 41293 (2001)). Par ailleurs, les souris transgéniques où le gène de la 11βHSD1 a été surexprimé dans les tissus
adipeux présentent un phénotype similaire à celui du syndrome métabolique humain (Masuzaki et al., Science 294, 2166 (2001)). Il est à noter que le phénotype observé existe sans une augmentation du total des glucocorticoides circulants, mais est induit par l'augmentation spécifique de glucocorticoides actifs dans les dépôts adipeux.

Par ailleurs, de nouvelles classes d'inhibiteurs spécifiques de la 11βHSD1, sont apparus récemment :
- des arylsulfonamidothiazoles ont montré qu'ils amélioraient la sensibilité à l'insuline et réduisaient le niveau de glucose dans le sang de souris présentant une hyperglycémie (Barf et al., J. Med. Chem. 45, 3813 (2002)). De plus, dans une étude récente, il a été montré que ce type de composés réduisait la prise de nourriture ainsi que la prise de poids chez des souris obèses (Wang et Coll. Diabetologia 49, 1333 (2006*)).*
- des triazoles ont montré qu'ils amélioraient le syndrome métabolique et ralentissaient la progression de l'athérosclérose chez des souris (Hermanowski-Vosatka et al., J. Exp. Med. 202, 517 (2005)).

### B. Cognition et démence

Les problèmes cognitifs légers sont des phénomènes communs chez les personnes âgées et peuvent conduire finalement à la progression de la démence. Autant dans le cas d'animaux que d'humains âgés, les différences inter-individuelles pour les fonctions cognitives générales ont été reliées aux différences d'exposition à long terme aux glucocorticoïdes (Lupien et al., Nat. Neurosci. 1, 69, (1998)). Par ailleurs, la dérégulation de l'axe HPA (hypothalamo-hypophysio-surrénalien)) résultant dans l'exposition chronique aux glucocorticoïdes de certaines sous-régions du cerveau a été proposée comme contribuant au déclin des fonctions cognitives (Mc Ewen et al., Curr. Opin. Neurobiol. 5, 205, 1995). La 11βHSD1 est abondante dans le cerveau et est exprimée dans de nombreuses sous régions incluant l'hypothalamus, le cortex frontal et le cerebellum (Sandeep et al., Proc. Natl. Acad. Sci. 101, 6734 (2004)). Les souris déficientes en 11βHSD1 sont protégées contre les dysfonctionnements de l'hypothalamus associés aux glucocorticoïdes qui sont rattachés à la vieillesse (Yau et al., Proc. Natl. Acad. Sci. 98, 4716, (2001)). De plus, dans des études chez l'homme, il a été montré que l'administration de la carbenoxolone améliore la fluidité verbale et la mémoire verbale chez les personnes âgées (Yau et al., Proc. Natl. Acad. Sci. 98, 4716 (2001), Sandeep et al., Proc. Natl. Acad. Sci. 101, 6734 (2004)). Finalement, l'utilisation d'inhibiteurs sélectifs de la 11βHSD1 de type triazole a montré qu'ils prolongeaient la rétention de la mémoire chez des souris âgées (Rocha et al., Abstract 231 ACS meeting, Atlanta, 26-30 Mars 2006).

### C. Pression intra-oculaire

Les glucocorticoïdes peuvent être utilisés par voies topique ou systémique pour une grande variété de pathologies de l'ophtalmologie clinique. Une complication particulière de ces traitements est le glaucome induit par l'utilisation de corticostéroïdes. Cette pathologie est caractérisée par l'augmentation de la pression intra-oculaire (PIO). Dans les cas les plus graves et pour les formes non traitées, la PIO peut conduire à une perte de champ de vision partielle et éventuellement à une perte totale de la vision. La PIO est le résultat d'un déséquilibre entre la production d'humeur aqueuse et son drainage. L'humeur aqueuse est produite dans les cellules épithéliales non-pigmentées et le drainage est réalisé au travers des cellules du réseau trabéculaire. La 11βSD1 est localisée dans les cellules épithéliales non-pigmentées et sa fonction est clairement l'amplification de l'activité des glucocorticoïdes dans ces cellules (Stokes et al., Invest. Ophthalmol. Vis. Sci. 41, 1629 (2000)). Cette notion est confirmée par l'observation que la concentration en cortisol libre est fortement excédentaire par rapport à la cortisone dans l'humeur aqueuse (ratio 14/1). L'activité fonctionnelle de la 11βHSD1 dans les yeux a été évaluée en étudiant l'action de la carbenoxolone chez des volontaires sains. Après sept jours de traitement à la carbenoxolone, la PIO est réduite de 18% (Rauz et al., Invest. Ophtamol. Vis. Sci. 42, 2037 (2001)). L'inhibition de la 11βHSD1 dans les yeux est donc prédite comme réduisant la concentration locale en glucocorticoïdes et la PIO, produisant un effet bénéfique dans le traitement du glaucome et d'autres désordres de la vision.

### D. Hypertension

Les substances hypertensives issues des adipocytes comme la leptine et l'angiotensinogène ont été proposées comme étant des éléments clés dans les pathologies d'hypertension reliées à l'obésité (Wajchenberg et al., Endocr. Rev. 21, 697 (2000)). La leptine qui est sécrétée en excès chez les souris aP2-11βHSD1 transgéniques (Masuzaki et al., J. Clinical Invest. 112, 83 (2003)), peut activer différents réseaux de systèmes neuronaux sympathiques, incluant ceux qui régulent la pression artérielle (Matsuzawa et al., Acad. Sci. 892, 146 (1999)). De plus, le système rénine-angiotensine (SRA) a été identifié comme étant une vole déterminante dans la variation de la pression artérielle. L'angiotensinogène, qui est produit dans le foie et le tissu adipeux, est un substrat-clé pour la rénine et est à l'origine de l'activation du SRA. Le niveau plasmatique en angiotensiogène est significativement élevé dans les souris aP2-11βHSD1 transgéniques, comme le sont ceux de l'angiotensine Il et de l'aldostérone (Masuzaki et al., J. Clinical Invest. 112, 83 (2003)) ; ces éléments conduisent à l'élévation de la pression artérielle. Le traitement de ces souris par de faibles doses d'un antagoniste du récepteur de l'angiotensine If abolit cette hypertension (Masuzaki et al., J. Clinical Invest. 112, 83 (2003)). Ces informations illustrent l'importance de l'activation locale des glucocorticoïdes dans le tissu adipeux et le foie, et suggère que cette hypertension puisse être causée ou exacerbée par l'activité de la 11pHSD1 dans ces tissus. L'inhibition de la 11βHSD1 et la réduction du niveau de glucocorticoïdes dans le tissu adipeux et/ou dans le foie est donc prédit comme ayant un rôle bénéfique pour le traitement de l'hypertension et des pathologies cardiovasculaires associées.

### E. Ostéoporose

Le développement du squelette et les fonctions osseuses sont aussi régulées par l'action des glucocorticoïdes. La 11βHSD1 est présente dans les ostéoclastes et ostéoblastes. Le traitement de volontaires sains par la carbenoxolone a montré une diminution des marqueurs de résorption osseuse sans changement dans les marqueurs de formation des os (Cooper et al., Bone, 27, 375 (2000)). L'inhibition de la 11βHSD1 et la réduction du niveau de glucocorticoïdes dans les os pourraient donc être utilisées comme un mécanisme de protection dans le traitement de l'ostéoporose.

Le document WO 03106456 décrit des molécules possédant une activité inhibitrice d'enzyme dipeptidyl- peptidase-IV.

Le document WO 2004089896 divulgue des amides bi- ou tri-cyclique substitués modulant l'activité de la 11βHSD1.

On a maintenant trouvé des dérivés d'urées de tropane qui modulent l'activité de la 11BHSD1.

La présente invention a pour objet des composés répondant à la formule (I) : 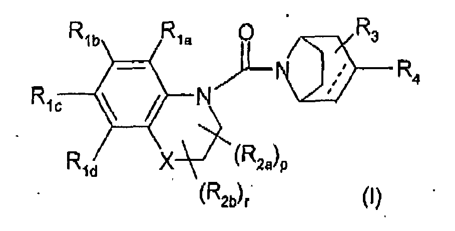 dans laquelle :
- X représente soit un atome de carbone, d'oxygène, de soufre ou d'azote soit le groupe -SO₂;
- la liaison en pointillés est une liaison simple ou une liaison double ;
- R_{1a,b},_{c,d} et R₂ₐ,_{b}, identiques ou différents, représentent chacun un atome d'hydrogène ou d'halogène ; un groupe (C₁-C₅) alkyle ; (C₁-C₅) alcoxy ; (C₁-C₅) halogénoalkyle , hydroxy; hydroxy-(C₁-C₅) alkyle, (C₁-C₅) alcoxy-(C₁-C₅) alkyle ; cyano ; un groupe -COOR₅ ; un groupe -NR₆R₇ ; un groupe -COOR₅ - (C₁-C₅) alkyle, un groupe -NR₆R₇- (C₁-C₅) alkyle, un groupe -CONR₆R₇, un groupe -CONR₆R₇-(C₁-C₅) alkyle , un groupe -SO₂NR₆R₇ ;
- (R₂ₐ)ₚ ou (R_{2b})ᵣ peuvent également former avec l'atome de carbone auquel il sont rattachés un groupe C=O ;
- R₃ représente un atome d'hydrogène, un atome de fluor ou un groupe (C₁-C₅) alkyle ; (C₁-C₅) alcoxy ; alcoxy-(C₁-C₅) alkyle ; hydroxy ; hydroxy-(C₁-C₅) alkyle ; (C₁-C₅) halogénoalkyle ; cyano ; un groupe -COOR₅; un groupe -NR₆R₇; un groupe -COOR₅ -(C₁-C₅) alkyle , un groupe -NR₆R₇-(C₁-C₅) alkyle, un groupe -CONR₆R₇, un groupe -CONR₆R₇-(C₁-C₅) alkyle.
- R₄ représente :
   o un atome d'hydrogène, un groupe (C₁-C₅) alkyle ;
   o un groupe (C₃-C₆) cycloalkyle ;
   o un hétérocyle ;
   o un groupe aryle mono- ou bi-cyclique ayant de 5 à 10 atomes de carbone ;
   o un groupe hétéroaryle mono- ou bi-cyclique ayant de 2 à 9 atomes de carbone ;
- le groupe aryle ou hétéroaryle étant éventuellement substitué par 1 à 4 substituants choisis parmi les atomes d'halogène, les groupes (C₁-C₅) alkyle ; (C₁-C₅) alcoxy ; (C₁-C₅) halogénoalkyle ; hydroxy ; hydroxy-(C₁-C₅) alkyle, (C₁-C₅) alcoxy-(C₁-C₅) alkyle; cyano; phényle éventuellement substitué; benzoyle éventuellement substitué;
   -COOR₅ ; 1-NR₆R₇ ; un groupe -COOR₅ -(C1-C5) alkyle, un groupe -NR₆R₇- (C₁-C₅) alkyle, un groupe -CONR₆R₇, un groupe -CONR₆R₇-(C₁-C₅) alkyle , un groupe -SO₂NR₆R₇, un groupe -NR₆-COR₅ ;
- p et r, identiques ou différents, sont des nombres entiers égaux à 1 ou 2;
- R₅ représente un atome d'hydrogène, un groupe (C₁-C₅) alkyle ; un groupe (C₃-C₆) cycloalkyle ;
- R₆ et R₇, identiques ou différents, représentent chacun un atome d'hydrogène, un groupe (C₁-C₅) alkyle ; un groupe (C₃-C₆) cycloalkyle ; (C₁-C₅) alkylcarbonyl ; hydroxyméthyl (C₁-C₅) alkyle ; (C₁-C₅)alcoxyméthyl (C₁-C₅) alkyle ; un groupe aryle, un groupe -SO₂-R₅ ou peuvent former ensemble avec l'atome d'azote auquel ils sont rattachés un hétérocycle éventuellement substitué.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister sous forme de stéréoisomères endo / exo. Ces stéréoisomères endo/exo ainsi que leurs mélanges font partie de l'invention.

Les composés de formule (I) peuvent exister à l'état de bases ou salifiés par des acides ou des bases, notamment des acides ou des bases pharmaceutiquement acceptables. De tels sels d'addition font partie de l'invention.

Ces sels sont avantageusement préparés - avec des acides pharmaceutiquement acceptables, mais les sels d'autres acides utiles, par exemple, pour la purification ou l'isolement des composés de formule (I), font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, et sauf mention différente dans le texte, on entend par :
- atome d'halogène : un fluor, un chlore, un brome ou un iode ;
- un groupe (C₁-C₅) alkyle : un groupe aliphatique saturé linéaire ou ramifié ayant 1 à 5 atomes de carbone successifs. A titre d'exemples, on peut citer les groupes méthyle, éthyle, propyle, isopropyle, butyle, isobutyle, tertbutyle, pentyle, etc ;
- un groupe (C₃-C₆) cycloalkyle : un groupe alkyle cyclique ayant 3 à 6 atomes de carbone. A titre d'exemples, on peut citer les groupes cyclopropyle, méthylcyclopropyle, cyclobutyle, cyclopentyle, cyclohexyle, etc ;
- un groupe (C₁-C₅) alcoxy : un radical -O- (C₁-C₅) alkyle où le groupe (C₁-C₅) alkyle est tel que précédemment défini ;
- un groupe aryle : un groupe aromatique mono- ou bi-cyclique comprenant entre 5 à 10 atomes de carbone. A titre d'exemples de groupes aryles, on peut citer le groupe phényle, le groupe thiophène, le groupe furane ou le groupe naphtalène.
- un groupe hétéroaryle : un groupe aromatique mono- ou bi-cyclique comprenant entre 5 et 9 atomes de carbone et comprenant entre 1 et 3 hétéroatomes, tels que l'azote, l'oxygène ou le soufre. A titre d'exemples de groupes hétéroaryles, on peut citer les groupes :

- pyridine
- pyrazine
- pyrimidine
- pyrazole
- oxadiazole
- thiazole
- imidazole
- benzothiophène
- quinoline
- indole
- un groupe (C₁-C₅) halogénoalkyle : un groupe (C₁-C₅) alkyle tel que défini ci-dessus substitué par 1 à 5 atomes d'halogène. On citera par exemple les groupes fluorométhyle, difluorométhyle, trifluorométhyle, trichlorométhyle ou encore pentafluoroéthyle.
- un hétérocycle: un groupe mono- ou bi-cyclique éventuellement fusionné
ou ponté comportant de 4 à 10 atomes dont un au moins est choisi parmi les atomes d'oxygène, d'azote ou de soufre. On citera par exemple les groupes 2,3-dihydrobenzofurane et 1,4-benzodioxane.
- un groupe "phényle éventuellement substitué", "benzyle éventuellement substitué", "hétérocycle éventuellement substitué" : un groupe phényle ou benzyle ou un hétérocycle qui sont éventuellement substitués par un ou plusieurs des groupes ci-après : les atomes d'halogène, les groupes (C₁-C₅) alkyle ; (C₁-C₅) alcoxy ; (C₁-C₅) halogénoalkyle ; hydroxy ; hydroxy-(C₁-C₅) alkyle, (C₁-C₅) alcoxy-(C₁-C₅) alkyle ; cyano ; phényle , benzyle, -COOR₅ ; -NR₆R₇ ; un groupe -COOR₅-(C₁-C₅) alkyle, un groupe -NR₆R₇-(C₁-C₅) alkyle, un groupe -CONR₆R₇, un groupe -CONR₆R₇-(C₁-C₅) alkyle , un groupe - SO₂NR₆R₇.
- R_{1a,b,c,d} désigne les groupes R₁ₐ, R_{1b}, R_{1c} et R_{1d} et R₂ₐ,_{b}, désigne les groupes R₂ₐ et R_{2b}.

Parmi les composés de formule (I) objets de l'invention, on peut citer un sous-groupe de composés particulièrement préférés dans lesquels X est le carbone ou l'oxygène, R₁ à R₇, X, p, r et la liaison en pointillés étant tels que définis ci-dessus.

Parmi ces derniers composés, des composés particulièrement préférés de l'invention sont des composés de formule (I) dans lesquels:
P et r représentent 1;
la liaison en pointillés représente un liaison simple ou double ;
R_{1a,b,c,d} représentent l'hydrogène, ou l'un des groupes R_{1a,b,c,d} est un halogène et les autres sont l'hydrogène ;
R_{2a,b} représentent l'hydrogène ou l'un des groupes R_{2a,b} est un groupe (C₁-C₅) alkyle, de préférence le méthyle et l'autre groupe R_{2a,b} est l'hydrogène ;
R₃ représente l'hydrogène ;
R₄ en position 4 est choisi parmi les aryles ou hétéroaryles suivants :
   - pyridine
   - phényle
   - pyrazole

Un autre groupe de composés particulièrement préférés au sens de l'invention correspond aux dérivés de formule (I) dans laquelle X représente l'atome de carbone et la liaison en pointillés représente un liaison double, R₄ est un phényle, ou une pyridine, R_{1a,b,c,d}, R₂ₐ,_{b}, R₃, R₅ à R₇, p et r étant définis tels que définis ci-dessus.

Un autre groupe de composés particulièrement préférés au sens de l'invention correspond aux dérivés de formule (I) dans laquelle X représente l'atome d'oxygène et la liaison en pointillé représente un liaison simple, R₄ est un phényle, ou une pyridine, R_{1a,b,c,d}, R_{2a,b}, R₃, R₅ à R₇. p et r étant tels que définis ci-dessus.

Un autre groupe de composés particulièrement préférés au sens de l'invention correspond aux dérivés de formule (I) dans laquelle X représente l'atome de carbone et la liaison en pointillé représente un liaison simple, R₄ est un phényle, une pyridine, ou un pyrazole, R_{1a,b,c,d}, R_{2a,b}, R₃, R₅ à R₇, p et r étant tels que définis ci-dessus.

Parmi les composés de formule (I) selon l'invention, on peut mentionner :
(3,4-Dihydro-2H-quinolin-1-yl)-((1S,3S,5R)-3-pyridin-4-yl-8-aza-bicyclo[3.2.1]oct-8-yl)-methanone
(3,4-Dihydro-2H-quinolin-1-yl)-(3-pyridin-3-yl-8-aza-bicyclo[3.2.1]oct-2-en-8-yl)-methanone
(3,4-Dihydro-2H-quinolin-1-yl)-(3-pyridin-4-yl-8-aza-bicyclo[3.2.1]oct-2-en-8-yl)-methanone
(3,4-Dihydro-2H-quinolin-1-yl)-((1S,5R)-3-pyridin-3-yl-8-aza-bicyclo[3.2.1]oct-8-yl)-methanone
(3,4-Dihydro-2H-quinolin-1-yl)-(3-pyridin-2-yl-8-aza-bicyclo[3.2.1]oct-8-yl)-methanone
(3,4-Dihydro-2H-quinolin-1-yl)-(3-pyridin-2-yl-8-aza-bicyclo[3.2.1]oct-8-yl)-methanone
(3,4-Dihydro-2H-quinolin-1-yl)-(3-phenyl-8-aza-bicyclo[3.2.1]oct-8-yl)-methanone
(3,4-Dihydro-2H-quinolin-1-yl)-[3-(6-fluoro-pyridin-3-yl)-8-aza-bicyclo[3.2.1]oct-8-yl]-methanone
[3-(4-Chloro-phenyl)-8-aza-bicyclo[3.2.1]oct-2-en-8-yl]-(3,4-dihydro-2H-quinolin-1-yl)-methanone
(3,4-Dihydro-2H-quinolin-1-yl)-[3-(2-fluoro-phenyl)-8-aza-bicyclo[3.2.1]oct-2-en-8-yl]-methanone
(3,4-Dihydro-2H-quinolin-1-yl)-[3-(2-ethyl-phenyl)-8-aza-bicyclo[3.2.1]oct-2-en-8-yl]-methanone
2-[8-(3,4-Dihydro-2H-quinoline-1-carbonyl)-8-aza-bicyclo[3.2.1]oct-2-en-3-yl]-benzonitrile
(3,4-Dihydro-2H-quinolin-1-yl)-[3-(2-fluoro-pyridin-3-yl)-8-aza-bicyclo[3.2.1]oct-2-en-8-yl]-methanone
[3-(2-Chloro-phenyl)-8-aza-bicyclo[3.2.1]oct-2-en-8-yl]-(3,4-dihydro-2H-quinolin-1-yl)-methanone
(3,4-Dihydro-2H-quinolin-1-yl)-[3-(2-trifluoromethyl-phenyl)-8-aza-bicyclo[3.2.1]oct-2-en-8-yl]-methanone
5-[8-(3,4-Dihydro-2H-quinoline-1-carbonyl)-8-aza-bicyclo[3.2.1]oct-2-en-3-yl]-thiophene-2-carbonitrile
(3-Benzo[b]thiophen-2-yl-8-aza-bicyclo[3.2.1]oct-2-en-8-yl)-(3,4-dihydro-2H-quinolin-1-yl)-methanone
(3,4-Dihydro-2H-quinolin-1-yl)-(3-m-tolyl-8-aza-bicyclo[3.2.1]oct-2-en-8-yl)-methanone
(3,4-Dihydro-2H-quinolin-1-yl)-[3-(4-isopropyl-phenyl)-8-aza-bicyclo[3.2.1]oct-2-en-8-yl]-methanone
4-[8-(3,4-Dihydro-2H-quinoline-1-carbonyl)-8-aza-bicyclo[3.2.1]oct-2-en-3-yl]-benzonitrile
(3,4-Dihydro-2H-quinolin-1-yl)-[3-(4-methoxy-phenyl)-8-aza-bicyclo[3.2.1]oct-2-en-8-yl]-methanone
3-[8-(3,4-Dihydro-2H-quinoline-1-carbonyl)-8-aza-bicyclo[3.2.1]oct-2-en-3-yl]-benzonitrile
(3,4-Dihydro-2H-quinolin-1-yl)-[3-(2-methoxy-phenyl)-8-aza-bicyclo[3.2.1]oct-2-en-8-yl]-methanone
2-[8-(3,4-Dihydro-2H-quinoline-1-carbonyl)-8-aza-bicyclo[3.2.1]oct-2-en-3-yl]-benzamide
[3-(2-Chloro-thiophen-3-yl)-8-aza-bicyclo[3.2.1]oct-2-en-8-yl]-(3,4-dihydro-2H-quinolin-1-yl)-methanone
(3,4-Dihydro-2H-quinolin-1-yl)-[3-(4-ethyl-phenyl)-8-aza-bicyclo[3.2.1]oct-2-en-8-yl]-methanone
[3-(2,3-Dihydro-benzo[1,4]dioxin-6-yl)-8-aza-bicyclo[3.2.1]oct-2-en-8-yl]-(3,4-dihydro-2H-quinolin-1-yl)-methanone
(3,4-Dihydro-2H-quinolin-1-yl)-[3-(1H-indolol-4-yl)-8-aza-bicyclo[3.2.1]oct-2-en-8-yl]-methanone
(3,4-Dihydro-2H-quinolin-1-yl)-(3-quinolin-5-yl-8-aza-bicyclo[3.2.1]oct-2-en-8-yl)-methanone
(3,4-Dihydro-2H-quinolin-1-yl)-[3-(6-methoxy-pyridin-3-yl)-8-aza-bicyclo[3.2.1]oct-2-en-8-yl]-methanone
(3,4-Dihydro-2H-quinolin-1-yl)-[3-(6-methoxy-pyridin-3-yl)-8-aza-bicyclo[3.2.1]oct-2-en-8-yl]-methanone
(3,4-Dihydro-2H-quinolin-1-yl)-[3-(3-fluoro-phenyl)-8-aza-bicyclo[3.2.1]oct-2-en-8-yl]-methanone
[3-(2,3-Dihydro-benzofuran-5-yl)-8-aza-bicyclo[3.2.1]oct-2-en-8-yl]-(3,4-dihydro-2H-quinolin-1-yl)-methanone
(3,4-Dihydro-2H-quinolin-1-yl)-[3-(4-trifluoromethyl-phenyl)-8-aza-bicyclo[3.2.1]oct-2-en-8-yl]-methanone
(3,4-Dihydro-2H-quinolin-1-yl)-[3-(4-fluoro-phenyl)-8-aza-bicyclo[3.2.1]oct-2-en-8-yl]-methanone
(3,4-Dihydro-2H-quinolin-1-yl)-(3-p-tolyl-8-aza-bicyclo[3.2.1]oct-2-en-8-yl)-methanone
(3,4-Dihydro-2H-quinolin-1-yl)-[3-(4-hydroxymethyl-phenyl)-8-aza-bicyclo[3.2.1]oct-2-en-8-yl]-methanone
N-{4-[8-(3,4-Dihydro-2H-quinoline-1-carbonyl)-8-aza-bicyclo[3.2.1]oct-2-en-3-yl]-phenyl}-acetamide
4-[8-(3,4-Dihydro-2H-quinoline-1-carbonyl)-8-aza-bicyclo[3.2.1]oct-2-en-3-yl]-N,N-dimethyl-benzamide
(3,4-Dihydro-2H-quinolin-1-yl)-[3-(1H-indol-6-yl)-8-aza-bicyclo[3.2.1]oct-2-en-8-yl]-methanone
(3,4-Dihydro-2H-quinolin-1-yl)-[3-(2-methoxy-pyridin-3-yl)-8-aza-bicyclo[3.2.1]oct-2-en-8-yl]-methanone
(3,4-Dihydro-2H-quinolin-1-yl)-(3-isoquinolin-4-yl-8-aza-bicyclo[3.2.1]oct-2-en-8-yl)-methanone
[3-(2-Chloro-pyridin-4-yl)-8-aza-bicyclo[3.2.1]oct-2-en-8-yl]-(3,4-dihydro-2H-quinolin-1-yl)-methanone
(3,4-Dihydro-2H-quinolin-1-yl)-(3-naphthalen-1-yl-8-aza-bicyclo[3.2.1]oct-2-en-8-yl)-methanone
(3,4-Dihydro-2H-quinolin-1-yl)-(3-thiophen-3-yl-8-aza-bicyclo[3.2.1]oct-2-en-8-yl)-methanone
[3-(4-Amino-phenyl)-8-aza-bicyclo[3.2.1]oct-2-en-8-yl]-(3,4-dihydro-2H-quinolin-1-yl)-methanone
(3,4-Dihydro-2H-quinolin-1-yl)-[3-(6-fluoro-pyridin-3-yl)-8-aza-bicyclo[3.2.1]oct-2-en-8-yl]-methanone
(3,4-Dihydro-2H-quinolin-1-yl)-[3-(6-fluoro-2-methyl-pyridin-3-yl)-8-aza-bicyclo[3.2.1]oct-2-en-8-yl]-methanone
[3-(2-Chloro-6-methyl-pyridin-3-yl)-8-aza-bicyclo[3.2.1]oct-2-en-8-yl]-(3,4-dihydro-2H-quinolin-1-yl)-methanone
(3,4-Dihydro-2H-quinolin-1-yl)-[3-(2,6-dimethoxy-pyridin-3-yl)-8-aza-bicyclo[3.2.1]oct-2-en-8-yl]-methanone
(3,4-Dihydro-2H-quinolin-1-yl)-(3-isoquinolin-5-yl-8-aza-bicyclo[3.2.1]oct-2-en-8-yl)-methanone
(3,4-Dihydro-2H-quinolin-1-yl)-[3-(8-methyl-quinolin-5-yl)-8-aza-bicyclo[3.2.1]oct-2-en-8-yl]-methanone
(3,4-Dihydro-2H-quinolin-1-yl)-[3-(6-ethoxy-pyridin-3-yl)-8-aza-bicyclo[3.2.1]oct-2-en-8-yl]-methanone
(3,4-Dihydro-2H-quinolin-1-yl)-[3-(2-ethoxy-pyridin-3-yl)-8-aza-bicyclo[3.2.1]oct-2-en-8-yl]-methanone
[3-(2,6-Difluoro-pyridin-3-yl)-8-aza-bicyclo[3.2.1]oct-2-en-8-yl]-(3,4-dihydro-2H-quinolin-1-yl)-methanone
[3-(5-Chloro-2-methoxy-pyridin-4-yl)-8-aza-bicyclo[3.2.1]oct-2-en-8-yl]-(3,4-dihydro-2H-quinolin-1-yl)-methanone
[3-(2,5-Dichloro-pyridin-3-yl)-8-aza-bicyclo[3.2.1]oct-2-en-8-yl]-(3,4-dihydro-2H-quinolin-1-yl)-méthanone
(3-Benzo[b]thiophen-5-yl-8-aza-bicyclo[3.2.1]oct-2-en-8-yl)-(3,4-dihydro-2H-quinolin-1-yl)-methanone
(3-Benzo[b]thiophen-7-yl-8-aza-bicyclo[3.2.1]oct-2-en-8-yl)-(3,4-dihydro-2H-quinolin-1-yl)-methanone
(3,4-Dihydro-2H-quinolin-1-yl)-[3-(1-methyl-1H-indol-2-yl)-8-aza-bicyclo[3.2.1]oct-2-en-8-yl]-methanone
[3-(6-Chloro-4-methyl-pyridin-3-yl)-8-aza-bicyclo[3.2.1]oct-2-en-8-yl]-(3,4-dihydro-2H-quinolin-1-yl)-methanone
(3,4-Dihydro-2H-quinolin-1-yl)-(3-pyridin-3-yl-8-aza-bicyclo[3.2.1]oct-2-en-8-yl)-methanone
(3,4-Dihydro-2H-quinolin-1-yl)-(3-pyridin-4-yl-8-aza-bicyclo[3.2.1]oct-2-en-8-yl)-methanone
(3,4-Dihydro-2H-quinolin-1-yl)-[3-(2H-pyrazol-3-yl)-8-aza-bicyclo[3.2.1]oct-8-yl]-methanone
(3,4-Dihydro-2H-quinolin-1-yl)-[3-(2H-pyrazol-3-yl)-8-aza-bicyclo[3.2.1]oct-8-yl]-methanone

Dans ce qui suit, on entend par groupe protecteur (Pg) un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans « Protective Groups in Organic Synthesis », Green et al., 3rd Edition (John Wiley & Sons, Inc., New York).

On entend par groupe partant (Lg), dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, paranitrophényle etc. Des exemples de groupes partants ainsi que des références pour leur préparation sont donnés dans « Advances in Organic Chemistry », J. March, 3rd Edition, Wiley Interscience, p. 310-316.

Conformément à l'invention, on peut préparer les composés de formule générale (I) selon les procédés ci-après. Dans le cas où X représente un atome d'azote, il doit être substitué soit par un groupe R₂ₐ,_{b} (différent de H) soit par un groupe protecteur Pg tel que défini précédemment.

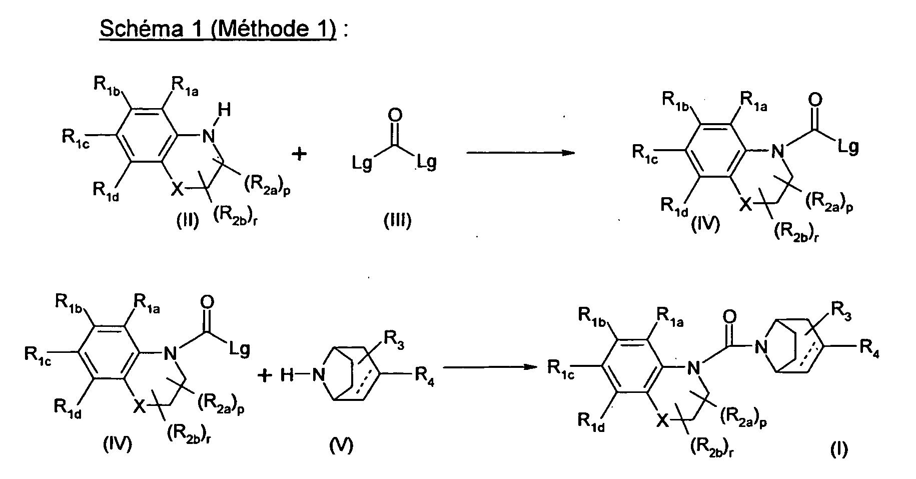

Dans le schéma 1, les composés de formule (IV) peuvent être préparés par réaction entre les intermédiaires de formule (II) et un carbonyle de formule (III) présentant deux groupes partant Lg (par exemple un atome de chlore, un groupe trichlorométhoxy, un groupe para-nitrophényle, un groupe imidazole, ou méthyl-imidazolium) en présence d'une base comme la triéthylamine ou la diisopropylamine dans un solvant tel que le dichlométhane, le tétrahydrofurane à une température variant de la température ambiante à 80°C. Les composés de formules (I) sont obtenus par couplage entre les dérivés activés (IV) et les amines (V) en présence ou non d'une base comme la triéthylamine ou le carbonate de potassium dans un solvant tel que le tétrahydrofurane, le dichlorométhane, l'acétonitrile ou l'eau, à une température variant de la température ambiante à 100°C.

Les hétérocycles de formule générale (II) sont disponibles commercialement ou peuvent être préparés par des méthodes décrites dans la littérature («Comprehensive heterocyclic chemistry », Katritzky et al., 2rd Edition (Pergamon press)).

Les hétérocycles de formule générale (V) sont disponibles commercialement ou peuvent être préparés par des méthodes décrites dans la littérature (Sikazwe et col. Biorg.Med. Chem. Lett 14, 5739 (2004) ; Gilbert et col. Biorg.Med. Chem. Lett 14, 515 (2004) ; Lu et col. Biorg.Med. Chem. Lett 13, 1817 (2003),)

Le schéma 2 détaille une synthèse des composés de formule (VI) dans lesquels la liaison en pointillé est une liaison double et R₄ représente un groupe aryle ou hétéroaryle tels que définis précédemment.

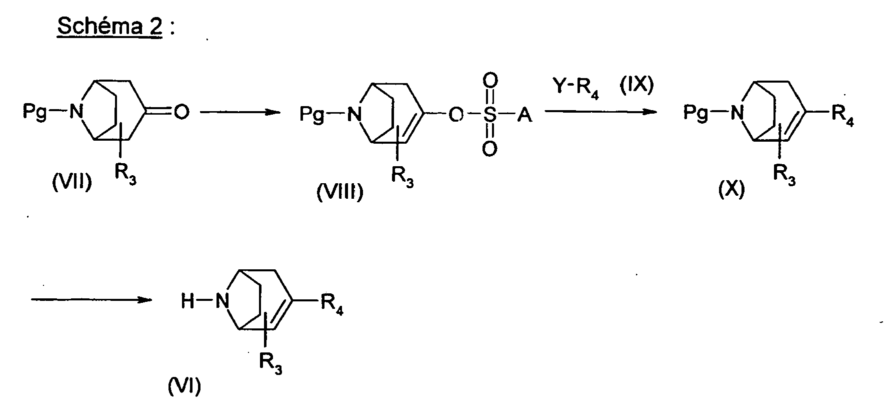

Dans le schéma 2, les hétérocycles (VIII), dont la fonction amine est protégée par un groupement protecteur Pg (par exemple un groupement Boc ou Fmoc) présentant un groupe vinyle sulfonate-A (par exemple A peut être un groupe trifluorométhyle, un groupe nonafluorobutyle), peuvent être préparés par transformation des cétones (VII) avec un agent de sulfonatation tel que l'anhydride trifluorosulfonique ou le N-phényltrifluorométhanesulfonimide en présence d'une base comme le diisopropyle amidure de lithium ou l'hexaméthyle disilazane de lithium dans un solvant tel que le tétrahydrofurane ou l'éthylèneglycol diméthyl éther à une température variant de -78°C à la température ambiante. Les hétérocycles (X) sont obtenus par couplage organométallique entre un composés (VIII) et un composé (IX) où Y est un dérivé du bore (par exemple un acide boronique ou un ester boronique), de l'étain (par exemple un groupe tri n-butyle étain) ou un atome d'halogène (par exemple le brome ou l'iode) en présence d'un dérivé métallique approprié (par exemple des dérivés du palladium, du zinc ou du cuivre) en présence d'une base ou non telle que le carbonate de potassium, le fluorure de potassium ou phosphate de sodium dans un solvant ou mélange de solvants, tels que le dioxane, l'éthylène glycol diméthyléther, le toluène ou l'eau, à une température variant de la température ambiante à 120°C. Dans une dernière étape, les amines de formule (VI) sont obtenues par déprotection de la fonction amine des composés de formule (X), par des méthodes choisies parmi celles connues de l'homme du métier. Elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Le schéma 3 détaille une synthèse des composés de formule (XI) dans lesquels la liaison en pointillé est une liaison simple et R₄ représente un groupe aryle ou hétéroaryle tels que définis ci-avant.

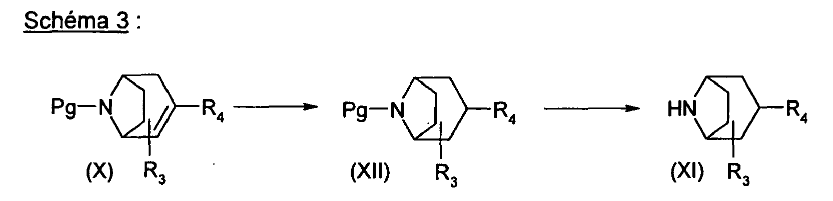

Dans le schéma 3, les hétérocycles (XII) sont obtenus par hydrogénation de la double liaison des hétérocycles (X) avec un catalyseur métallique approprié dans le méthanol ou l'éthanol. Dans la deuxième étape, les amines de formule (XI) sont obtenues par déprotection de la fonction amine des composés de formule (XII) par des méthodes choisies parmi celles connues de l'homme du métier. Elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C.

Eventuellement dans le schéma 4, le mélange des stéréoisomères endo(XIII)/exo(XIV) peut être séparé au moyen d'une chromatographie flash, d'une chromatographie liquide haute pression ou d'une recristallisation, autrement il est utilisé tel que et dénommé mélange (XI).

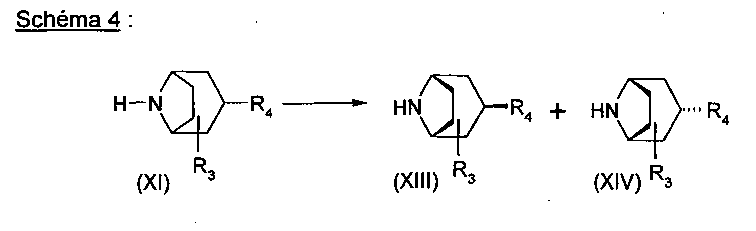

Le schéma 5 présente une voie de préparation alternative des composés de formule (I) dans lesquels la liaison en pointillés est une liaison simple et R₄ représente un groupe aryle ou hétéroaryle tels que définis précédemment, ces composés sont appelés ci-après composés de formule (XV). Dans le cas où X représente un atome d'azote, il doit être substitué soit par un groupe R_{2a,b} (différent de H) soit par un groupe protecteur Pg tel que défini précédemment. 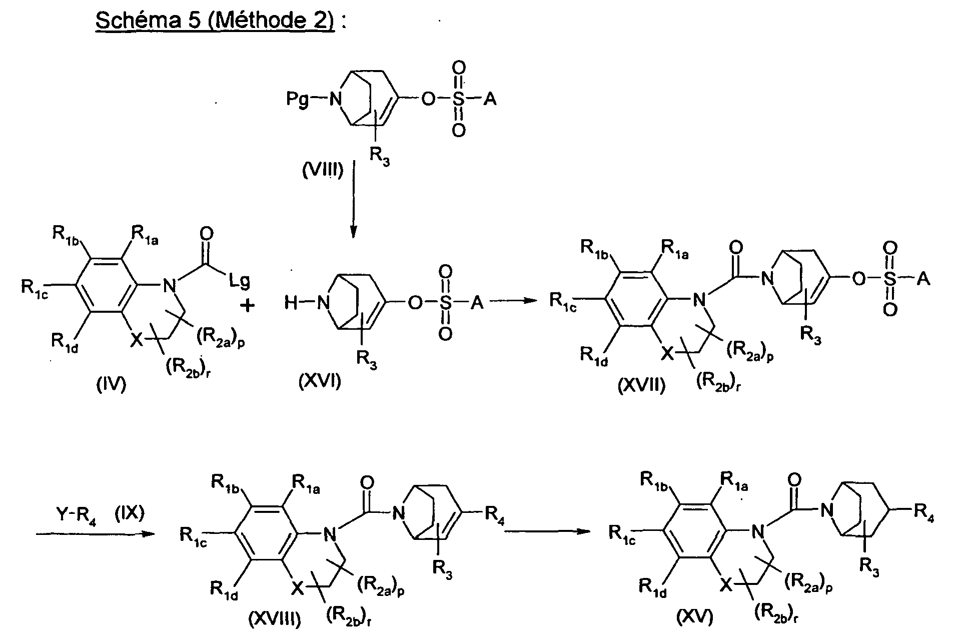 Dans le schéma 5, les amines (XVI) sont obtenues par déprotection de la fonction amine des composés de formule (VIII), par des méthodes choisies parmi celles connues de l'homme du métier. Elles comprennent entre autres l'utilisation d'acide trifluoroacétique ou d'acide chlorhydrique dans le dichlorométhane, le dioxane, le tétrahydrofurane ou le diéthyléther dans le cas d'une protection par un groupement Boc, et de pipéridine pour un groupement Fmoc, à des températures variant de -10 à 100°C. Les composés de formule (XVII) sont obtenus par couplage entre les dérivés activés (IV) et les amines (XVI) en présence ou non d'une base comme la triéthylamine ou le carbonate de potassium dans un solvant tel que le tétrahydrofurane, le dichlorométhane, l'acétonitrile ou l'eau, à une température variant de la température ambiante et 100°C. Dans l'étape suivante, les hétérocycles (XVIII) sont obtenus par couplage organométallique entre un composé (XVII) et un composé (IX) où Y est un dérivé du bore (par exemple un acide boronique ou un ester boronique), de l'étain (par exemple un groupe tri n-butyle étain) ou un atome d'halogène (par exemple le brome ou l'iode) en présence d'un dérivé métallique approprié (par exemple un dérivé du palladium, du zinc ou du cuivre) en présence d'une base ou non telle que le carbonate de potassium, le fluorure de potassium ou phosphate de sodium dans un solvant ou mélange de solvant tels que le dioxane, l'éthylène glycol diméthyléther, le toluène ou l'eau, à une température variant de la température ambiante à 120°C. Dans une dernière étape, la double liaison des hétérocycles (XVIII) est hydrogénée avec un métal approprié dans le méthanol ou l'éthanol pour conduire aux dérivés (XV).

Eventuellement dans le schéma 6, le mélange des stéréoisomères endo(XIX)/exo(XX) peut être séparé au moyen d'une chromatographie flash, d'une chromatographie liquide haute pression ou d'une recristallisation, autrement il est utilisé tel que et dénommé mélange (XV).

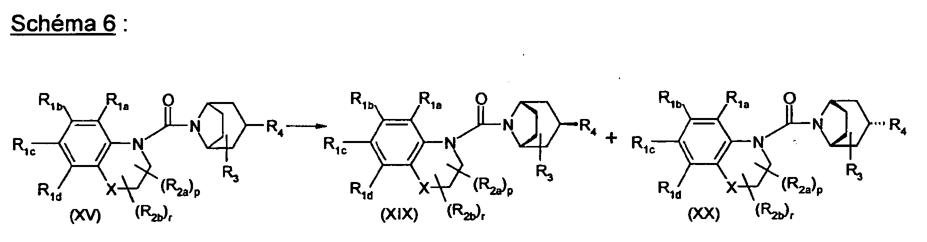

Les exemples suivants décrivent la préparation de certains composés conformes à l'invention. Ces exemples ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 : Chlorhydrate du 1-[(3-pyridin-3-yl-8-azabicyclo[3.2.1]oct-8-yl)carbonyl]-1,2,3,4-tétrahydroquinoline (Composé 4)

### 1.1 : tert-butyl 3-{[(trifluorométhyl)sulfonyl]oxy}-8-azabicyclo[3.2.1]oct-2-èn-8-carbo-xylate.

Dans un tricol de 500 ml sous azote, on ajoute goutte à goutte 10 ml d'une solution 2,5 N de *n*-butyl lithium dans l'hexane à une solution de 3,73 ml de diisopropylamine dans 100 ml de tétrahydrofurane refroidi à -60°C. Après 1/4 d'heure d'agitation, on ajoute 5 g de N-*tert*-butyloxycarbonyl nortropinone dans le tétrahydrofurane (50 ml) à 0°C. Finalement, toujours à 0°C, on ajoute 8,32 g de N-phényltrifluorométhanesulfonimide. Après 24 heures d'agitation à température ambiante, on évapore le tétrahydrofurane et on purifie le produit par filtration rapide sur alumine en utilisant comme éluant un mélange d'heptane/acétate d'éthyle 2/1. On obtient 6,13 g de *tert*-butyl 3-{[(trifluorométhyl)sulfonyl]oxy}-8-azabicyclo[3.2.1]oct-2-èn-8-carboxylate.

M+H⁺=358

### 1.2 : Chlorhydrate du 8-azabicyclo[3.2.1]oct-2-èn-3-yl trifluorométhylsulfonate.

Dans un ballon de 100 ml sont placés 2,76 g de *tert*-butyl 3-{[(trifluorométhyl)sulfonyl]oxy}-8-azabicyclo[3.2.1]oct-2-èn-8-carboxylate dans 13 ml de dioxane. On ajoute ensuite doucement, 12,8 ml d'une solution d'acide chlorhydrique 4N dans le dioxane. Le mélange réactionnel est mis sous agitation pendant 3h. Le dioxane est évaporé pour conduire à 2,27 g de chlorhydrate du 8-azabicyclo[3.2.1]oct-2-èn-3-yl trifluoro-méthylsuifonate.

M+H⁺=258

### 1.3 8-(3,4-dihydroquinolin-1(2H)-ylcarbonyl)-8-azabicyclo[3.2.1]oct-2-èn-3-yl trifluorométhanesulfonate.

Dans un tricol de 250 ml sous atmosphère d'azote est placé 1,13 g de 1;2,3,4-tétrahydroquinoline, 85 ml de dichlorométhane et 1,54 ml de triéthylamine. On ajoute à 0°C, 0,834 g de triphosgène, puis la réaction est laissée sous agitation à température ambiante pendant 4h. On rajoute ensuite 2,27 g de chlorhydrate du 8-azabicyclo[3.2.1]oct-2-èn-3-yl trifluorométhylsulfonate ainsi que 1,19 ml de triéthylamine, puis le mélange réactionnel est mis au reflux pour 18h. On ajoute 200 ml d'une solution aqueuse saturée de hydrogénocarbonate de sodium, puis la phase aqueuse est extraite trois fois au dichlorométhane. On rassemble les phases organiques, on sèche sur sulfate de sodium, et on évapore sous pression réduite. Le résidu est chromatographié sur gel de silice par un mélange d'heptane/acétate d'éthyle 8/2. On obtient 3,21 g de 8-(3,4-dihydroquinolin-1(2H)-ylcarbonyl)-8-azabicyclo[3.2.1]oct-2-èn-3-yl trifluorométhanesulfonate.

M+H⁺=417

### 1.4 : 1-[(3-pyridin-3-yl-8-azabicyclo[3.2.1]oct-2-èn-8-yl)carbonyl]-1,2,3,4-tétra-hydroquinoline

Dans un tube en verre de 10 ml est introduit 0,5 g de dihydroquinolin-1(2H)-ylcarbonyl)-8-azabicyclo[3.2.1]oct-2-èn-3-yl trifluorométhanesulfonate, 0,419 g de 3-(4,4,5,5-tétramethyl-1,3,2-dioxaborolan-2-yl)pyridine, 0,1 g de chlorure de lithium, 0,765 g de phosphate de potassium, 0,067 g de 2'-(diméthylamino)-2-biphényl-palladium(II) chlorure dinorbornylphosphine et 4,3 ml de dioxane. Le tube est scellé, puis chauffé à 100°C sous irradiation micro-ondes pendant 50 minutes. De l'eau et de l'acétate d'éthyle sont ajoutés. La phase aqueuse est extraite trois fois avec de l'acétate d'éthyle. On rassemble les phases organiques, on sèche sur sulfate de sodium et on évapore le solvant sous pression réduite. Le résidu est chromatographié sur gel de silice par un mélange d'heptane/acétate d'éthyle 3/7. On obtient 0,3 g de 1-[(3-pyridin-3-yl-8-azabicyclo[3.2.1]oct-2-èn-8-yl)carbonyl]-1,2,3,4-tétrahydroquinoline.

M+H⁺=346

### 1.5 : 1-[(3-pyridin-3-yl-8-azabicyclo[3.2.1]oct-8-yl)carbonyl]-1,2,3,4-tétra-hydroquinoline

Dans un réacteur haute pression, sous azote, 0,148 g de palladium sur charbon 5% est additionné à 0,240 g de la 1-[(3-pyridin-3-yl-8-azabicyclo[3.2.1]oct-2-èn-8-yl)carbonyl]-1,2,3,4-tétrahydroquinoline solubilisée dans 9 ml d'éthanol. Le mélange réactionnel est mis sous une pression de 3 atmosphères d'hydrogène, à 25°C, et agité mécaniquement pendant 15 heures. Le palladium est filtré sur papier Whatman et est lavé par du méthanol. Le solvant est évaporé, puis le résidu est chromatographié sur gel de silice avec un gradient d'éluant heptane/acétate d'éthyle (3/7) à heptane/acétate d'éthyle (2/8). On obtient 0,146 g de 1-[(3-pyridin-3-yl-8-azabicyclo[3.2.1]oct-8-yl)carbonyl]-1,2,3,4-tétrahydroquinoline.
M+H⁺=348,3

### 1.6 : Chlorhydrate du 1-[(3-pyridin-3-yl-8-azabicyclo[3.2.1]oct-8-yl)carbonyl]-1,2,3,4-tétrahydroquinoline

On dissout 0,146 g de 1-[(3-pyridin-3-yl-8-azabicyclo[3.2.1]oct-8-yl)carbonyl]-1,2,3,4-tétrahydroquinoline dans 6 ml de dichlorométhane et on ajoute 4,2 ml d'une solution d'acide chlorhydrique 4N dans le dioxane. Après évaporation, on reprend le résidu dans l'acétate d'éthyle. Le précipité est filtré puis séché sous vide. On obtient 0,122 g de chlorhydrate du 1-[(3-pyridin-3-yl-8-azabicyclo[3.2.1]oct-8-yl)carbonyl]-1,2,3,4-tétrahydroquinoline.

Point de fusion=218-220°C

M+H⁺=348,3

RMN ¹H (DMSO, 200MHz), δ(ppm) : 1,4-2 (m, 9H) ; 2,2-2,44 (m, 1H) ; 2,6-2,78 (t, 2H) ; 2,9-3,1 (m, 0,4H) ; 3,2-3,45 (m, 0,6H) ; 3,5-3,61 (m, 2H) ; 4,1 (sl, 2H) ; 6,78-6,98 (m, 1H) ; 7-7,2 (m,3H) ; 7,8-7,96 (m, 1H) ; 8,3-8,55 (m, 1H) ; 8,64-8,9 (m, 2H).

### Exemple 2 : Chlorhydrate du 1-{[(3-endo)-3-pyridin-2-yl-8-azabicyclo[3.2.1]oct-8-yl]carbonyl}-1,2,3,4-tétrahydroquinoline (Composé 5)

### 2.1 1-[(3-pyridin-2-yl-8-azabicyclo[3.2.1]oct-2-èn-8-yl)carbonyl]-1,2,3,4-tétrahydroquinoline

Dans un tube en verre de 80 ml on introduit 2,87 g de tri n-butyl étain 2-pyridine, 0,6 g de chlorure de lithium, 0,5 g de dihydroquinolin-1(2H)-ylcarbonyl)-8-aza- bicyclo[3.2.1]oct-2-èn-3-yl trifluorométhanesulfonate, 0,249 g de dichlorure de diphénylphosphine-palladium(II) et 19 ml de tétrahydrofurane. Le tube est scellé, puis chauffé à 140°C sous irradiation micro-onde pendant 1 heure. Le mélange réactionnel est filtré, puis de l'acétate d'éthyle et une solution aqueuse de fluorure de potassium à 0,5N sont ajoutés. Le mélange est ensuite filtré, puis la phase organique est séchée sur sulfate de sodium et on évapore le solvant sous pression réduite. Le résidu est chromatographié sur gel de silice par un mélange d'heptane/acétate d'éthyle. On obtient 0,73 g de 1-[(3-pyridin-2-yl-8-azabicyclo[3.2.1]oct-2-en-8-yl)carbonyl]-1,2,3,4-tétrahydroquinoline.

M+H⁺=346

### 2-2 1-{[(3-endo)-3-pyridin-2-yl-8-azabicyclo[3.2.1]oct-8-yl]carbonyl}-1,2,3,4-tétrahydroquinoline

Dans un réacteur haute pression, sous azote, 0,5 g de palladium sur charbon 5% est additionné à 0,81 g de la 1-[(3-pyridin-2-yl-8-azabicyclo[3.2.1]oct-2-èn-8-yl)carbonyl]-1,2,3,4-tétrahydroquinoline solubilisée dans 29 ml d'éthanol. Le mélange réactionnel est mis sous une pression de 3 atmosphères d'hydrogène, à 25°C, et agité mécaniquement pendant 7 heures. Le palladium est filtré sur papier Whatman et lavé par du méthanol. Le solvant est évaporé, puis le résidu est chromatographié sur gel de silice, gradient d'éluant d'heptane/acétate d'éthyle. On obtient 0,221g de 1-{[(3-endo)-3-pyridin-2-yl-8-azabicyclo[3.2.1]oct-8-yl]carbonyl}-1,2,3,4-tétrahydroquinoline et 0,141 g de 1-{[(3-*exo*)-3-pyridin-2-yl-8-azabicyclo[3.2.1]oct-8-yl]carbonyl}-1,2,3,4-tétrahydroquinoline.

M+H⁺=348,3

### 2.3 Chlorhydrate du 1-{[(3-endo)-3-pyndin-2-yl-8-azabicyclo[3.2.1]oct-8-yl]carbonyl}-1,2,3,4-tétrahydroquinoline

On dissout 0,221 g de 1-{[(3-*endo*)-3-pyridin-2-yl-8-azabicyclo[3.2.1]oct-8-yl]carbonyl}-1,2,3,4-tétrahydroquinoline dans 5,3 ml de dichlorométhane et on ajoute 6,4 ml d'une solution d'acide chlorhydrique 0,2N dans l'éther. Après évaporation, on reprend le résidu dans de l'acétate d'éthyle. Le précipité est filtré puis séché sous vide. On obtient 0,161 g de chlorhydrate du 1-{[(3-*endo*)-3-pyridin-2-yl-8-azabicyclo[3.2.1]oct-8-yl]carbonyl}-1,2,3,4-tétrahydroquinoline.

Point de fusion=161-163°C ;

M+H⁺=348,3 ;

RMN ¹H (CDCl₃, 200MHz), δ(ppm) : 1,36-1,56 (m, 2H); 1,67-1,8 (m, 2H); 1,85-2,1 (m, 4H); 2,24-2,4 (m, 2H); 2,7 (t, 2H); 3 (q, 1H) ; 3,62 (t, 2H) ; 4-4,12 (m,2H) ; 6,82 (t,1H) ; 6,93-7,09 (m, 3H) ; 7,15-7,3 (m, 2H) ; 7,43-7,59 (m, 1H) ; 8,4-8,5 (m, 1H).

### Exemple 3 : Chlorhydrate du 1-{[(3-exo)-3-pyridin-2-yl-8-azabicyclo[3.2.1]oct-8-yl]carbonyl}-1,2,3,4-tétrahydroquinoline (Composé 6)

On dissout 0,13 g de 1-{[(3-exo)-3-pyridin-2-yl-8-azabicyclo[3.2.1]oct-8-yl]carbonyl}-1,2,3,4-tétrahydroquinoline obtenue selon l'exemple 2 (partie 2.2) dans 3,1 ml de dichlorométhane et on ajoute 3,7 ml d'une solution d'acide chlorhydrique 0,2N dans de l'éther. Après évaporation, on reprend le résidu dans de l'acétate d'éthyle. Le précipité est filtré puis séché sous vide. On obtient 0,101 g de chlorhydrate du 1-{[(3-*exo*)-3-pyridin-2-yl-8-azabicyclo[3.2.1]oct-8-yl]carbonyl}-1,2,3,4-tétrahydroquinoline.

Point de fusion=178-180°C ;

M+H⁺=348,3 ;

RMN ¹H (CDCl₃, 200MHz), δ(ppm) : 1,65-2 (m, 10H) ; 2,7 (t, 2H) ; 3,09-3,3 (m, 1H) ; 3,2 (t, 2H) ; 4-4,2 (t, 2H) ; 6,84 (t, 1H) ; 6,95-7,23 (m, 5H) ; 7,5-7,67 (m, 1H) ; 8,45 (d, 1H).

### Exemple 4 : Chlorhydrate du 1-[(4-pyridin-3-yl-8-azabicyclo[3.2.1]oct-8-yl)carbonyl]-1 ,2,3,4-tétrahydroquinoline (Composé 1)

### 4.1 tert-butyl 3-pyridin-4-yl-8-azabicyclo[3.2.1]oct-2-èn-8-carboxylate

Dans un tube en verre de 80 ml on introduit 1 g de tert-butyl 3-{[(trifluorométhyl)sulfonyl]oxy}-8-azabicyclo[3.2.1]oct-2-ène-8-carboxylate, 0,975 g de 4-(4,4,5,5-tétraméthyl-1,3,2-dioxaborolan-2-yl)pyridine, 0,24 g de chlorure de lithium, 1,78 g de phosphate de potassium, 0,157 g de 2'-(diméthylamino)-2-biphényl-palladium(II) chlorure dinorbornylphosphine et 10 ml de dioxane. Le tube est scellé, puis chauffé à 100°C sous irradiation micro-onde pendant 30 minutes. De l'eau et de l'acétate d'éthyle sont ajoutés. La phase aqueuse est extraite trois fois avec de l'acétate d'éthyle. On rassemble les phases organiques, on sèche sur sulfate de sodium et on évapore le solvant sous pression réduite. Le résidu est chromatographié sur gel de silice par un mélange d'heptane/acétate d'éthyle. On obtient 0,458 g de tert-butyl 3-pyridin-4-yl-8-azabicyclo[3.2.1]oct-2-ène-8-carboxylate.

M+H⁺=387

### 4.2 tert-butyl 3-pyridin-4-yl-8-azabicyclo[3.2.1]octane-8-carboxylate

Dans un réacteur haute pression, sous azote, 0,17 g de palladium sur charbon 5% est additionné à 0,46 g de *tert*-butyl 3-pyridin-4-yl-8-azabicyclo[3.2.1]oct-2-ène-8-carboxylate solubilisé dans 20 ml de méthanol. Le mélange réactionnel est mis sous une pression de 3 atmosphères d'hydrogène à 25°C, et agité mécaniquement pendant deux heures et demi. Le palladium est filtré sur papier Whatman et lavé par du méthanol. Le solvant est évaporé et on obtient 0,46 g de *tert*-butyl 3-pyridin-4-yl-8-azabicyclo[3.2.1]octane-8-carboxylate.

M+H⁺=289

### 4.3 Dichlorhydrate de 3-pyridin-4-yl-8-azabicyclo[3.2.1]octane

Dans un ballon de 10 ml on place 2,76 g de *tert*-butyl 3-pyridin-4-yl-8-azabicyclo[3.2.1]octane-8-carboxylate et 4 ml d'une solution d'acide chlorhydrique 4N dans du dioxane. Le mélange réactionnel est mis sous agitation pendant 1 heure et demi. Le dioxane est évaporé et on obtient à 0,341 g de dichlorhydrate de 3-pyridin-4-yl-8-azabicyclo[3.2.1 ]octane.

M+H⁺=189

### 4.4 1-[(4-pyridin-3-yl-8-azabicyclo[3.2.1]oct-8-yl)carbonyl]-1,2,3,4-tétrahydroquinoline

Dans un ballon de 50 ml sous atmosphère d'azote on place 0,241 g de 1,2,3,4-tétrahydroquinoline, 20 ml de dichlorométhane et 0,24 ml de triéthylamine. On ajoute à 0°C, 0,178 g de triphosgène, puis le mélange réactionnel est laissé sous agitation à température ambiante pendant 3h. On rajoute ensuite 0,34 g de dichlorhydrate de 3-pyridin-4-yl-8-azabicyclo[3.2.1]octane ainsi que 1,19 ml de triéthylamine, puis le mélange réactionnel est mis au reflux pendant trois heures. On ajoute 200 ml d'une solution aqueuse saturée d'hydrogénocarbonate de sodium, puis la phase aqueuse est extraite trois fois avec du dichlorométhane. On rassemble les phases organiques, on sèche sur sulfate de sodium et on évapore sous pression réduite. Le résidu est chromatographié sur gel de silice par un mélange de dichlorométhane/méthanol 9/1. On obtient 0,04 g de 1-[(4-pyridin-3-yl-8-azabicyclo[3.2.1]oct-8-yl)carbonyl]-1,2,3,4-tétrahydroquinoline.

M+H⁺=348

### 4.5 Chlorhydrate du 1-[(4-pyridin-3-yl-8-azabicyclo[3.2.1]oct-8-yl)carbonyl]-1,2,3,4-tétrahydroquinoline

On dissout 0,04 g de 1-[(4-pyridin-3-yl-8-azabicyclo[3.2.1]oct-8-yl)carbonyl]-1,2,3,4-tétra-hydroquinoline dans 1,15 ml d'une solution d'acide chlorhydrique 0,2N dans de l'éther. Après évaporation, on reprend le résidu dans de l'acétate d'éthyle. Le précipité est filtré puis séché sous vide. On obtient 0,004 g de chlorhydrate du 1-[(4-pyridin-3-yl-8-azabicyclo[3.2.1]oct-8-yl)carbonyl]-1,2,3,4-tétrahydroquinoline.

Point de fusion=216-222°C ;

M+H⁺=348,2 ;

RMN ¹H (DMSO, 200MHz), δ(ppm) : 1,65-1,95 (m, 10H) ; 2,65 (t, 2H) ; 3,2-3,4(m, 1H) ; 3,45 (t, 2H) ; 4,05 (sl, 1H) ; 6,75-6,9 (m, 1H) ; 6,95-7,2 (m, 2H) ; 7,78 (d, 2H) ; 8,64 (d, 2H).

Le tableau qui suit illustre les structures chimiques et les propriétés physiques de quelques exemples de composés selon l'invention. Dans ce tableau :
- dans la colonne « sel », « - » représente un composé sous forme de base libre, alors que « HCl » représente un composé sous forme de chlorhydrate.

### Tableau

| | | | | | | | | | | | | | |
|---|---|---|---|---|---|---|---|---|---|---|---|---|---|
| 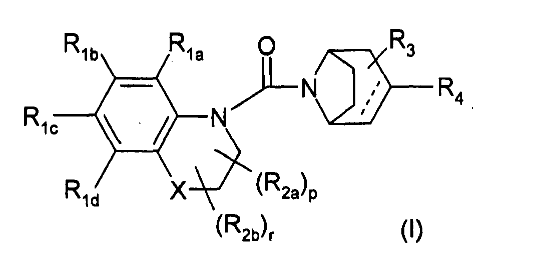 | | | | | | | | | | | | | |
| dans les exemples 1 à 64 ci-après : p = r = 1 et R₂ₐ = R_{2b} = H | | | | | | | | | | | | | |

| **N°** | **X** | ---- | **R₁ₐ** | **R_{1b}** | **R_{1c}** | **R_{1d}** | **R₃** | **Endo/exo** | **R₄** | **Sel** | **PF (°C)** | **LCUVMS MASS (amu)** | **Méthode** |
|---|---|---|---|---|---|---|---|---|---|---|---|---|---|
| **1** | C | ――― | H | H | H | H | H | Endo/exo | 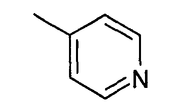 | HCl | 211-216 | | 1 |
| **2** | C | ――― | H | H | H | H | H | - | 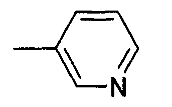 | HCl | 150-176 | | 2 |
| **3** | C | ――― | H | H | H | H | H | - | 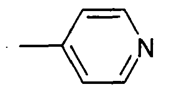 | | 218-22 | | 2 |
| **4** | C | ――― | H | H | H | H | H | Endo/exo | 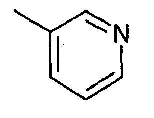 | HCl | 218-222 | | 2 |
| **5** | C | ――― | H | H | H | H | H | endo | 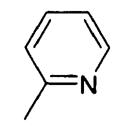 | HCl | 161-163 | | 2 |
| **6** | C | ――― | H | H | H | H | H | Exo | 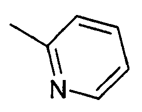 | HCl | 178-180 | | 2 |
| **7** | C | ――― | H | H | H | H | H | Endo/exo | 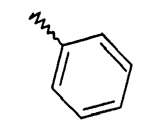 | - | 133-134 | | 2 |
| **8** | C | ――― | H | H | H | H | H | Endo/exo | 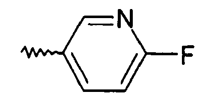 | HCl | 98-99 | | 2 |
| **9** | C | ――― | H | H | H | H | H | - | 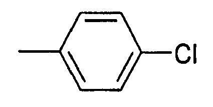 | - | - | 379,22 | 2 |
| **10** | C | ――― | H | H | H | H | H | - | 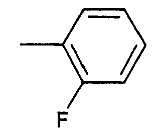 | - | - | 363,24 | 2 |
| **11** | C | ――― | H | H | H | H | H | - | 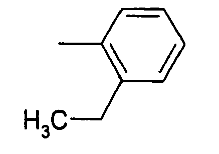 | - | - | 373,32 | 2 |
| **1**2 | C | ――― | H | H | H | H | H | - | 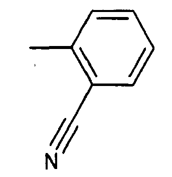 | - | - | 370,24 | 2 |
| **13** | C | ――― | H | H | H | H | H | - | 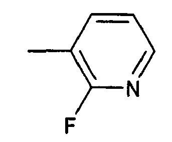 | - | - | 364,23 | 2 |
| **14** | C | ――― | H | H | H | H | H | - | 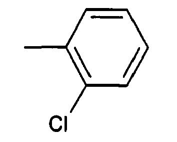 | - | - | 379,21 | 2 |
| **15** | C | ――― | H | H | H | H | H | - | 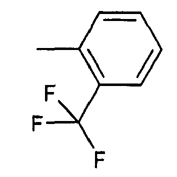 | - | - | 413,23 | 2 |
| **16** | C | ――― | H | H | H | H | H | - | 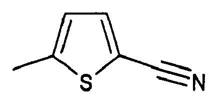 | - | - | 376,24 | 2 |
| **17** | C | ――― | H | H | H | H | H | - | 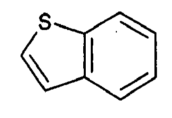 | - | - | 401,28 | 2 |
| **18** | C | ――― | H | H | H | H | H | - | 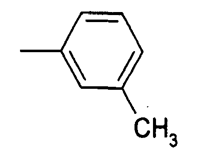 | - | - | 359,29 | 2 |
| **19** | C | ――― | H | H | H | H | H | - | 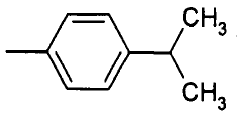 | - | - | 387,34 | 2 |
| **20** | C | ――― | H | H | H | H | H | - | 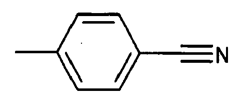 | - | - | 370,27 | 2 |
| **21** | C | ――― | H | H | H | H | H | - | 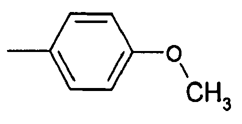 | - | - | 375,26 | 2 |
| **22** | C | ――― | H | H | H | H | H | - | 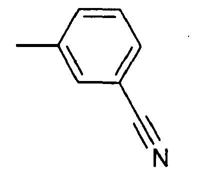 | - | - | 370,21 | 2 |
| **23** | C | ――― | H | H | H | H | H | - | 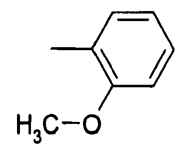 | - | - | 375,26 | 2 |
| **24** | C | ――― | H | H | H | H | H | - | 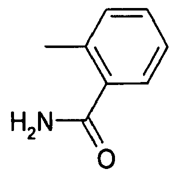 | - | - | 388,21 | 2 |
| **25** | C | ――― | H | H | H | H | H | - | 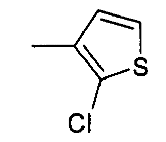 | - | - | 385,16 | 2 |
| **26** | C | ――― | H | H | H | H | H | - | 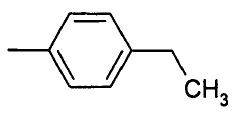 | - | - | 373,27 | 2 |
| **27** | C | ――― | H | H | H | H | H | - | 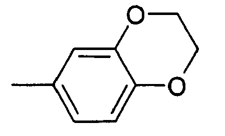 | - | - | 403,23 | 2 |
| **28** | C | ――― | H | H | H | H | H | - | 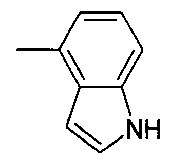 | - | - | 384,23 | 2 |
| **29** | C | ――― | H | H | H | H | H | - | 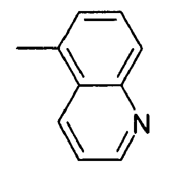 | TFA | - | 396,25 | 2 |
| **30** | C | ――― | H | H | H | H | H | - | 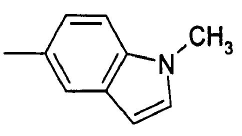 | TFA | - | 398,24 | 2 |
| **31** | C | ――― | H | H | H | H | H | - | 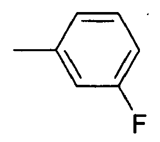 | - | - | 363,17 | 2 |
| **32** | C | ――― | H | H | H | H | H | - | 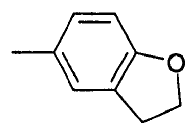 | - | - | 387,22 | 2 |
| **33** | C | ――― | H | H | H | H | H | - | 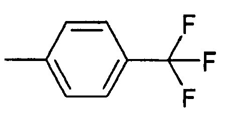 | - | - | 413,19 | 2 |
| **34** | C | ――― | H | H | H | H | H | - | 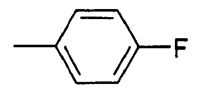 | - | - | 363,17 | 2 |
| **35** | C | ――― | H | H | H | H | H | - | 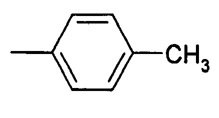 | - | - | 359,18 | 2 |
| **36** | C | ――― | H | H | H | H | H | - | 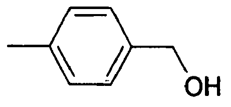 | - | - | 375,26 | 2 |
| **37** | C | ――― | H | H | H | H | H | - | 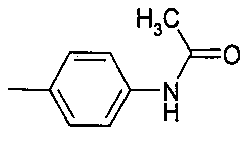 | - | - | 402,3 | 2 |
| **38** | C | ――― | H | H | H | H | H | - | 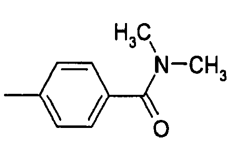 | - | - | 416,26 | 2 |
| **39** | C | ――― | H | H | H | H | H | - | 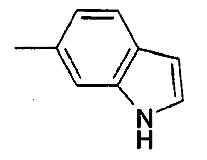 | TFA | - | 384,23 | 2 |
| **40** | C | ――― | H | H | H | H | H | - | 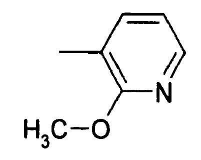 | TFA | - | 376,19 | 2 |
| **41** | C | ――― | H | H | H | H | H | - | 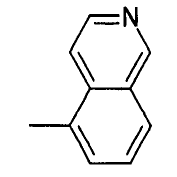 | TFA | - | 396,23 | 2 |
| **42** | C | ――― | H | H | H | H | H | - | 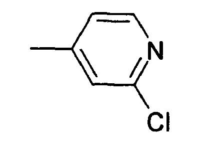 | - | - | 380,5 | 2 |
| **43** | C | ――― | H | H | H | H | H | - | 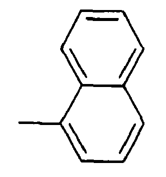 | - | - | 395,25 | 2 |
| **44** | C | ――― | H | H | H | H | H | - | 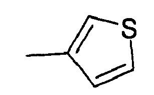 | - | - | 351,15 | 2 |
| **45** | C | ――― | H | H | H | H | H | - | 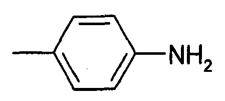 | TFA | - | 360,26 | 2 |
| **46** | C | ――― | H | H | H | H | H | - | 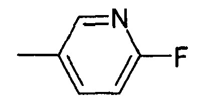 | - | - | 364,18 | 2 |
| **47** | C | ――― | H | H | H | H | H | - | 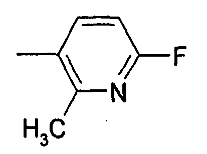 | - | - | 378,21 | 2 |
| **48** | C | ――― | H | H | H | H | H | - | 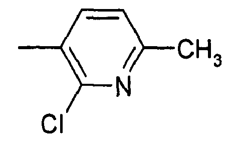 | - | - | 394,18 | 2 |
| **49** | C | ――― | H | H | H | H | H | - | 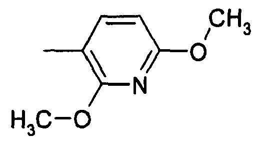 | - | - | 406,17 | 2 |
| **50** | C | ――― | H | H | H | H | H | - | 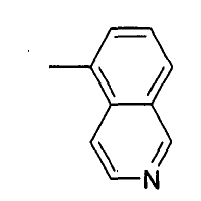 | TFA | - | 396,22 | 2 |
| **51** | C | ――― | H | H | H | H | H | - | 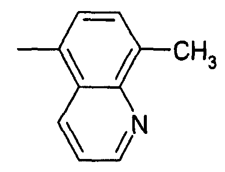 | TFA | - | 410,2 | 2 |
| **52** | C | ――― | H | H | H | H | H | - | 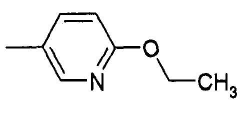 | TFA | - | 390,2 | 2 |
| **53** | C | ――― | H | H | H | H | H | - | 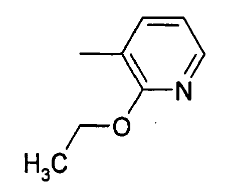 | TFA | - | 390,17 | 2 |
| **54** | C | ――― | H | H | H | H | H | - | 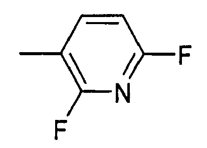 | - | - | 382,19 | 2 |
| **55** | C | ――― | H | H | H | H | H | - | 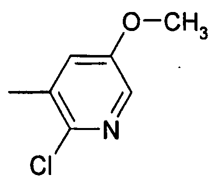 | - | - | 410,18 | 2 |
| **56** | C | ――― | H | H | H | H | H | - | 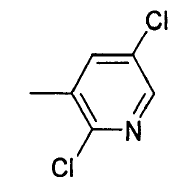 | - | - | 414,13 | 2 |
| **57** | C | ――― | H | H | H | H | H | - | 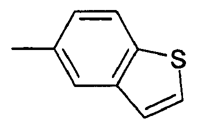 | - | - | 401,18 | 2 |
| **58** | C | ――― | H | H | H | H | H | - | 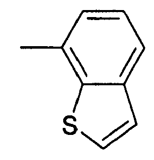 | - | - | 401,17 | 2 |
| **59** | C | ――― | H | H | H | H | H | - | 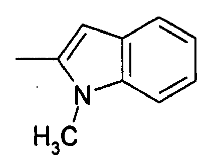 | - | - | 398,24 | 2 |
| **60** | C | ――― | H | H | H | H | H | - | 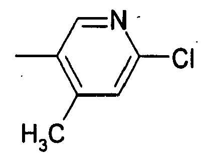 | - | - | 394,16 | 2 |
| **61** | C | ――― | H | H | H | H | H | - | 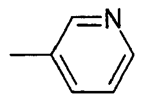 | - | - | 346 | 2 |
| **62** | C | ――― | H | H | H | H | H | - | 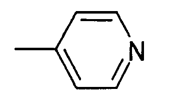 | - | - | 346,2 | 2 |
| **63** | C | ――― | H | H | H | H | H | endo | 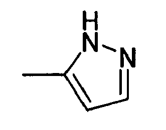 | - | 146-66 | | 2 |
| **64** | C | ――― | H | H | H | H | H | exo | 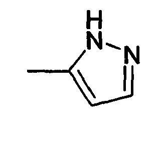 | - | 70-81°C | | 2 |

Les composés selon l'invention ont fait l'objet d'essais pharmacologiques permettant de déterminer leur effet inhibiteur de l'enzyme 11beta-HSD1 qui est une enzyme qui intervient dans le métabolisme des lipides ou le métabolisme du glucose.

Ces essais ont consisté à mesurer l'activité inhibitrice *in vitro* des composés de l'invention grâce à un test SPA (Scintillation Proximity Assay) en format 384 puits. La protéine 11bêta-HSD1 recombinante a été produite en levure *S.cerevisiae.* La réaction a été réalisée en incubant l'enzyme en présence de ³H-cortisone et de NADPH, en absence ou en présence de concentration croissante d'inhibiteur. Des billes SPA couplées à un anticorps anti-souris, préincubées avec un anticorps anti-cortisol, ont permis de mesurer la quantité de cortisol formé au cours de la réaction.

L'activité inhibitrice vis-à-vis de l'enzyme 11 bêta HSD1 est donnée par la concentration qui inhibe 50% de l'activité de 11 bêta-HSD1 (CI₅₀).

Les CI₅₀ des composés selon l'invention sont inférieures à 1 µM. Par exemple, les CI₅₀ des composés n° 4, 7, 13, 28 et 55 sont respectivement de 0,019 µM, 0,004 µM, 0,122 µM, 0,19 µM et 0,534 µM.

Il apparaît donc que les composés selon l'invention ont une activité inhibitrice de l'enzyme 11 béta-HSD1. Les composés selon l'invention peuvent donc être utilisés pour la préparation de médicaments, en particulier de médicaments inhibiteurs de l'enzyme 11béta-HSD1.

Ainsi, selon un autre de ses aspects, l'invention a pour objet des médicaments qui comprennent un composé de formule (I), ou un sel d'addition de ce dernier à un acide pharmaceutiquement acceptable, ou encore un hydrate ou un solvat du composé de formule (I).

Ces médicaments trouvent leur emploi en thérapeutique, notamment dans le traitement de l'obésité, des diabètes, de la résistance à l'insuline, du syndrome métabolique, du syndrome de Cushing, de l'hypertension, de l'athérosclérose, de la cognition et de la démence, des glaucomes, de l'ostéoporose et de certaines maladies infectieuses en augmentant l'efficacité du système immunitaire.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, Intra-veineuse, topique, locale, Intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculalre, intranasale, par inhalation, les formes d'administration topique, transdermique, sous-cutanée, intramusculaire ou intraveineuse, les formes d'administration rectale et les implants. Pour l'application topique, on peut utiliser les composés selon invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Dans la cadre d'une méthode de traitement des pathologies ci-dessus indiquées il est administré à un patient, une dose efficace d'un composé selon l'invention, ou un de ses sels pharmaceutiquement acceptables ou hydrates ou solvats.

## Revendications

1. Composé répondant à la formule (I) 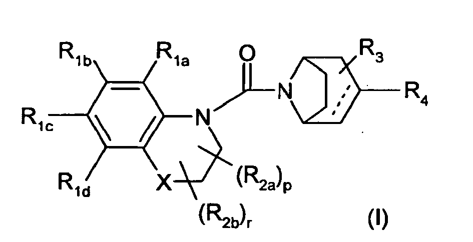 dans laquelle :
- X représente soit un atome de carbone, d'oxygène, de soufre ou d'azote soit le groupe SO₂ ;
- la liaison en pointillés est une liaison simple ou une liaison double
- R_{1a,b,c,d} et R_{2a,b,} identiques ou différents, représentent chacun un atome d'hydrogène ou d'halogène ; un groupe (C₁-C₅) alkyle ; (C₁-C₅) alcoxy ; (C₁-C₅) halogenoalkyle, hydroxy; hydroxy-(C₁-C₅) alkyle, (C₁-C₅) alcoxy-(C₁-C₅) alkyle ; cyano ; un groupe -COOR₅ ; un groupe -NR₆R₇; un groupe -COOR₅ -(C₁-C₅) alkyle, un groupe -NR₆R₇₋ (C₁-C₅) alkyle, un groupe -CONR₆R₇, un groupe -CONR₆R₇-(C₁-C₅) alkyle, un groupe -SO₂NR₆R₇;
- (R₂ₐ)ₚ ou (R_{2b})ᵣ peuvent également former avec l'atome de carbone auquel ils sont rattachés un groupe C=O ;
- R₃ représente un atome d'hydrogène, un atome de fluor ou un groupe (C₁-C₅) alkyle ; (C₁-C₅) alcoxy ; alcoxy-(C₁-C₅) alkyle ; hydroxy ; hydroxy-(C₁-C₅) alkyle ; (C₁-C₅) halogénoalkyle ; cyano ; un groupe -COOR₅; un groupe -NR₆R₇; un groupe -COOR₅ -(C₁-C₅) alkyle , un groupe NR₆R₇- (C₁-C₅) alkyle, un groupe-CONR₆R₇, un groupe -CONR₆R₇-(C₁-C₅) alkyle ;
- R₄ représente :
o un atome d'hydrogène, un groupe (C₁-C₅) alkyle ;
o un groupe (C₃-C₆) cycloalkyle ;
o un hétérocyle ;
o un groupe aryle mono ou bi-cyclique ayant de 5 à 10 atomes de carbone ;
o un groupe hétéroaryle mono ou bi-cyclique ayant de 2 à 9 atomes de carbone ;
le groupe aryle ou hétéroaryle étant éventuellement substitué par 1 à 4 substituants choisis parmi les atomes d'halogène, les groupes (C₁-C₅) alkyle ; (C₁-C₅) alcoxy ; (C₁-C₅) halogenoalkyle ; hydroxy ; hydroxy-(C₁-C₅) alkyle, (C₁-C₅) alcoxy-(C₁-C₅) alkyle ; cyano ; phényle éventuellement substitué; benzyle éventuellement substitué; -COOR₅ ; -NR₆R₇; un groupe - COOR₅ -(C1-C5) alkyle, un groupe NR₆R₇₋ (C₁-C₅) alkyle, un groupe -CONR₆R₇, un groupe-CONR₆R₇-(C₁-C₅) alkyle, un groupe -SO₂NR₆R₇, un groupe -NR₆-COR_{5.}
- p et r, identiques ou différents, sont des nombres entiers égaux à 1 ou 2 ;
- R₅ représente un atome d'hydrogène, un groupe (C₁-C₅) alkyle ; un groupe (C₃-C₆) cycloalkyle ;
- R₆ et R₇, identiques ou différents, représentent chacun un atome d'hydrogène, un groupe (C₁-C₅) alkyle; un groupe (C₃-C₆) cycloalkyle ; (C₁-C₅) alkylcarbonyl ; hydroxymethyl (C₁-C₅) alkyle; (C₁-C₅)alcoxymethyl (C₁-C₅) alkyle; un groupe aryle ; un groupe -SO₂-R₅ ou peuvent former ensemble avec l'atome d'azote auquel ils sont rattachés un hétérocycle éventuellement substitué ;
leurs sels, solvats et hydrates ainsi que leurs énantiomères et diastéréoisomères, y compris les mélanges racémiques.

2. Composés selon la revendication 1, dans lesquels dans lesquels X est le carbone ou l'oxygène, R₁ à R₇, X, p, r et la liaison en pointillés sont tels que définis dans la revendication 1.

3. Composés de formule (I) selon la revendication 2, dans lesquels:
p et r représentent 1 ;
la liaison en pointillés représente un liaison simple ou double ;
R_{1a,b,c,d} représentent l'hydrogène, ou l'un des groupes R_{1a,b,c,d} est un halogène et les autres groupes R_{1a,b,c,d} sont l'hydrogène ;
R_{2a,b} représentent l'hydrogène ou l'un des groupes R_{2a,b} est un groupe (C₁-C₅) alkyle, de préférence le méthyle et l'autre groupe est l'hydrogène ;
R₃ représente l'hydrogène ;
R₄ en position 4 est choisi parmi les aryles ou hétéroaryles suivants :
- pyridine
- phényle
- pyrazole

4. Composés de formule (I) selon la revendication 2 dans lesquels X représente l'atome de carbone et la liaison en pointillés représente un liaison double, R₄ est un phényle, ou une pyridine, R_{1a,b,c,d}, R_{2a,b}, R₃, R₅ à R₇, p et r étant tels que définis dans la revendication 1.

5. Composés de formule (I) selon la revendication 2, dans lesquels X représente l'atome d'oxygène et la liaison en pointillés représente un liaison simple, R₄ est un phényle, ou une pyridine, R_{1a,b,c,d}, R_{2a,b}, R₅ à R₇, p et r étant tels que définis dans la revendication 1.

6. Composés de formule (I) selon la revendication 2, dans lesquels X représente l'atome de carbone et la liaison en pointillés représente un liaison simple, R₄ est un phényle, une pyridine ou un pyrazole, R_{1a,b,c,d}, R_{2a,b}, R₅ à R₇, p et r étant tels que définis dans la revendication 1.

7. Procédé de préparation d'un composé de formule (I) selon l'une quelconque des revendications 1 à 6, **caractérisé en ce que** l'on fait réagir un composé de formule (IV) : 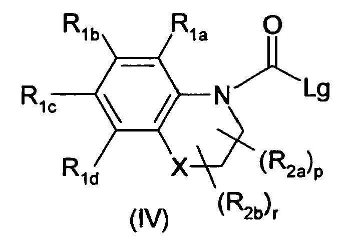 dans laquelle :
- R_{1a,b,c,d}, R_{2a,b} p et r sont tels que définis dans la revendication 1 pour les composés de formule (I) et
- Lg est un groupe partant ;
avec un composé de formule (V) 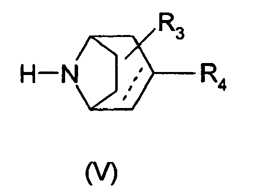 dans laquelle :
- R₃ et R₄ sont tels que définis dans la revendication 1 pour les composés de formule (I) ; et
éventuellement à transformer le composé ainsi obtenu en l'un de ses sels.

8. Médicament, **caractérisé en ce qu'**il comprend un composé de formule (I) selon l'une quelconque des revendications 1 à 6, ou un sel d'addition de ce composé à un acide pharmaceutiquement acceptable, ou encore un hydrate ou un solvat du composé de formule (I).

9. Composition pharmaceutique, **caractérisée en ce qu'**elle comprend un composé de formule (I) selon l'une quelconque des revendications 1 à 6, ou un sel pharmaceutiquement acceptable, un hydrate ou un solvat de ce composé, ainsi qu'au moins un excipient pharmaceutiquement acceptable.

10. Utilisation d'un composé de formule (I) selon l'une quelconque des revendications 1 à 6 pour la préparation d'un médicament destiné au traitement de l'obésité, des diabètes, de la résistance à l'insuline, du syndrome métabolique, du syndrome de Cushing, de l'hypertension, de l'athérosclérose, des troubles de la cognition et de la démence, des glaucomes, de l'ostéoporose et d'états pathologiques nécessitant l'activation du système immunitaire.

## Claims

1. Compound corresponding to formula (I) 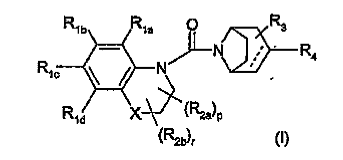 in which:
- X represents either a carbon, oxygen, sulfur or nitrogen atom or the -SO₂ group;
- the dashed-line bond is a single bond or a double bond;
- R_{1a,b,c,d} and R_{2a,b} which may be identical or different, each represent a hydrogen or halogen atom; a (C₁-C₅)alkyl; (C₁-C₅)alkoxy; (C₁-C₅)haloalkyl, hydroxyl; hydroxy(C₁-C₅)alkyl, (C₁-C₅)alkoxy(C₁-C₅)alkyl; a cyano group; a -COOR₅ group; an -NR₆R₇ group; a -COOR₅-(C₁-C₅)alkyl group, an -NR₆R₇-(C₁-C₅)alkyl group, a -CONR₆R₇ group, a CONR₆R₇-(C₁-C₅) alkyl group or an -SO₂NR₆R₇ group;
- (R₂ₐ)ₚ or (R_{2b})ᵣ may also form, with the carbon atom to which they are attached, a C=O group;
- R₃ represents a hydrogen atom, a fluorine atom, a (C₁-C₅)alkyl; (C₁-C₅) alkoxy; alkoxy(C₁-C₅)alkyl; hydroxyl; hydroxy(C₁-C₅)alkyl; (C₂-C₅)haloalkyl; a cyano group; a -COOR₅ group; an -NR₆R₇ group; a -COOR₅-(C₁-C₅)alkyl group, an -NR₆R₇-(C₁-C₅)alkyl group, a -CCNR₆R₇ group, or a -CONR₆R₇-(C₁-C₅)alkyl group;
- R₄ represents:
o a hydrogen atom or a (C₁-C₅)alkyl group;
o a (C₃-C₆)cycloalkyl group;
o a heterocycle;
o a monocyclic or bicyclic aryl group containing from 5 to 10 carbon atoms;
o a monocyclic or bicyclic heteroaryl group containing from 2 to 9 carbon atoms;
- the aryl or heteroaryl group being optionally substituted with 1 to 4 substituents chosen from halogen atoms and the groups: (C₁-C₅) alkyl; (C₁-C₅)alkoxy; (C₁-C₅) haloalkyl, hydroxyl; hydroxy(C₁-C₅)alkyl, (C₁-C₅)alkoxy(C₁-C₅)alkyl; cyano; optionally substituted phenyl; optionally substituted benzyl; -COOR₅; -NR₆R₇; a -COOR₅-(C₁-C₅)alkyl group, an -NR₆R₇-(C₁-C₅)alkyl group, a -CONR₆R₇ group, a -CONR₆R₇-(C₁-C₅)alkyl group, an -SO₂NR₆R₇ group, an -NR₆-COR₅ group;
- p and r, which may be identical or different, are integers equal to 1 or 2;
- R₅ represents a hydrogen atom, (C₁-C₅)alkyl group or a (C₃-C₆)cycloalkyl group;
- R₆ and R₇, which may be identical or different, each represent a hydrogen atom, a (C₁-C₅)alkyl group; a (C₃-C₆)cycloalkyl group; a (C₁-C₅) alkylcarbonyl group; a hydroxymethyl(C₁-C₅)alkyl group; a (C₁-C₅)alkoxymethyl (C₁-C₅)alkyl group; an aryl group or an -SO₂-R₅ group, or may form, together with the nitrogen atom to which they are attached, an optionally substituted heterocycle;
the salts, solvates and hydrates thereof and also the enantiomers and diasteroisomers thereof, including racemic mixtures.

2. Compound according to Claim 1, in which X is carbon or oxygen, and R₁ to R₇, X, p, r and the dashed-line bond are as defined in Claim 1.

3. Compound of formula (I) according to Claim 2, in which:
p and r represent 1;
the dashed-line bond represents a single or double bond;
R_{1a,b,c,d} represent hydrogen, or one of the groups R_{1a,b,c,d} is a halogen and the other groups R_{1a,b,c,d} are hydrogen;
R_{2a,b} represent hydrogen, or one of the groups R_{2a,b} is a (C₁-C₅)alkyl group, preferably methyl, and the other group is hydrogen;
R₃ represents hydrogen;
R₄ in position 4 is chosen from the following aryls or heteroaryls:
- pyridine
- phenyl
- pyrazole.

4. Compound of formula (I) according to Claim 2, in which X represents the carbon atom and the dashed-line bond represents a double bond, and R₄ is a phenyl or a pyridine, R_{1a,b,c,d}, R_{2a,b}, R₃, R₅ to R₇, p and r being as defined in Claim 1.

5. Compound of formula (I) according to Claim 2, in which X represents the oxygen atom and the dashed-line bond represents a single bond, and R₄ is a phenyl or a pyridine, R_{1a,b,c,d}, R_{2a,b}, R₅ to R₇, p and r being as defined in Claim 1.

6. Compound of formula (I) according to Claim 2, in which X represents the carbon atom and the dashed-line bond represents a single bond, and R₄ is a phenyl, a pyridine, or a pyrazole, R_{1a,b,c,d}, R_{2a,b}, R₅ to R₇, p and r being as defined in Claim 1.

7. Process for preparing a compound of formula (I) according to any one of Claims 1 to 6, **characterized in that** a compound of formula (IV): 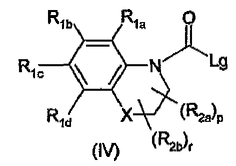 in which:
- R_{1a,b,c,d}, R_{2a,b}, p and r are as defined in Claim 1 for the compounds of formula (I), and
- Lg is a leaving group;
is reacted with a compound of formula (V) 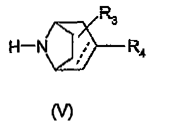 in which:
- R₃ and R₄ are as defined in Claim 1 for the compounds of formula (I); and
optionally, the compound thus obtained is converted to one of its salts.

8. Medicament, **characterized in that** it comprises a compound of formula (I) according to any one of Claims 1 to 6, or an addition salt of this compound with a pharmaceutically acceptable acid, or else a hydrate or a solvate of the compound of formula (I).

9. Pharmaceutical composition, **characterized in that** it comprises a compound of formula (I) according to any one of Claims 1 to 6, or a pharmaceutically acceptable salt, a hydrate or a solvate of this compound, and also at least one pharmaceutically acceptable excipient.

10. Use of a compound of formula (I) according to any one of Claims 1 to 6, for the preparation of a medicament for use in the treatment of obesity, diabetes, insulin resistance, metabolic syndrome, Cushing's syndrome, hypertension, atherosclerosis, cognitive disorders and dementia, glaucoma, osteoporosis and pathological conditions requiring activation of the immune system.

## Patentansprüche

1. Verbindungen der Formel (I) 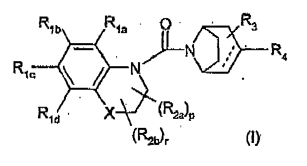 worin:
- X für ein Kohlenstoff-, Sauerstoff-, Schwefel- oder Stickstoffatom oder eine SO₂-Gruppe steht;
- die gestrichelte Linie für eine Einfachbindung oder eine Doppelbindung steht;
- R_{1a,b,c,d} und R_{2a,b} gleich oder verschieden sind und jeweils für ein Wasserstoff- oder Halogenatom; eine (C₁-C₅)-Alkylgruppe; eine (C₁-C₅)-Alkoxygruppe; eine (C₁-C₅)-Halogenalkylgruppe, eine Hydroxygruppe; eine Hydroxy-(C₁₋C₅)-alkylgruppe; eine (C₁-C₅)-Alkoxy-(C₁-C₅)-alkylgruppe; eine Cyanogruppe; eine -COOR₅-Gruppe; eine -NR₆R₇-Gruppe; eine -COOP₅- (C₁-C₅) -Alkylgruppe; eine -NR₆R₇-(C₁-C₅)-Alkyl-Gruppe; eine -CONR₆R₇-Gruppe, eine -CONR₆R₇-(C₁-C₅)-Alkylgruppe oder eine -SO₂NR₆R₇-Gruppe stehen;
- (R₂ₐ)ₚ oder (R_{2b})ᵣ auch mit dem Kohlenstoffatom, an das sie gebunden sind, eine C=O-Gruppe bilden können;
- R₃ für ein Wasserstoffatom, ein Fluoratom, eine (C₁-C₅)-Alkylgruppe; eine (C₁-C₅)-Alkoxygruppe; eine Alkoxy-(C₁-C₅)-alkylgruppe; eine Hydroxygruppe; eine Hydroxy-(C₁-C₅)-alkylgruppe; eine (C₁-C₅)-Halogenalkylgruppe; eine Cyanogruppe; eine -COOR₅-Gruppe; eine -NR₆R₇-Gruppe; eine -COOR₅-(C₁-C₅)-Alkylgruppe; eine -NR₆R₇-(C₁-C₅)-Alkyl-Gruppe; eine -CONR₅R₇-Gruppe oder eine -CONR₆R₇-(C₁-C₅)-Alkylgruppe steht;
- R₄ für:
ο ein Wasserstoffatom oder eine (C₁-C₅)-Alkylgruppe;
ο eine (C₃-C₆)-Cycloalkylgruppe;
ο einen Heterocyclus;
ο eine mono- oder bicyclische Arylgruppe mit 5 bis 10 Kohlenstoffatomen oder
ο eine mono- oder bicyclische Heteroarylgruppe mit 2 bis 9 Kohlenstoffatomen
steht; wobei die Aryl- oder Heteroarylgruppe gegebenenfalls durch 1 bis 4 unter Halogenatomen; (C₁-C₅) -Alkylgruppen; (C₁-C₅) -Alkoxygruppen; (C₁-C₅)-Halogenalkylgruppen; Hydroxygruppen; Hydroxy-(C₁-C₅)-alkylgruppen; (C₁-C₅)-Alkoxy-(C₁-C₅)-alkylgruppen; Cyanogruppen; gegebenenfalls substituiertem Phenyl; gegebenenfalls substituiertem Benzyl; -COOR₅; -NRₑR₇; einer -COOR₅-(C₁-C₅)-Alkylgruppe; einer NR₆R₇-(C₁-C₅)-Alkylgruppe; einer -CONR₆R₇-Gruppe; einer -CONR₆R₇-(C₁-C₅)-Alkylgruppe, einer -SO₂NR₆R₇-Gruppe und einer -NR₆-COR₅-Gruppe ausgewählte Substituenten substituiert ist;
- p und r gleich oder verschieden sind und für ganze Zahlen mit einem Wert von 1 oder 2 stehen;
- R₅ für ein Wasserstoffatom, eine (C₁-C₅)-Alkylgruppe oder eine (C₃-C₅)-Cycloalkylgruppe steht;
- R₆ und R₇ gleich oder verschieden sind und jeweils für ein Wasserstoffatom; eine (C₁-C₅)-Alkylgruppe; eine (C₃-C₆)-Cycloalkylgruppe; eine (C₁-C₅)-Alkylcarbonylgruppe; eine Hydroxymethyl-(C₁-C₅)-alkylgruppe; eine (C₁-C₅)-Alkoxymethyl-(C₁-C₅)-alkylgruppe; eine Arylgruppe oder eine -SO₂-R₅-Gruppe stehen oder zusammen mit dem Stickstoffatom, an das sie gebunden sind, einen gegebenenfalls substituierten Heterocyclus bilden können;
ihre Salze, Solvate und Hydrate sowie ihre Enantiomere und Diastereoisomere einschließlich racemischer Gemische.

2. Verbindungen nach Anspruch 1, worin X für Kohlenstoff oder Sauerstoff steht und R₁ bis R₇, X, p, r und die gestrichelte Bindung die in Anspruch 1 angegebene Bedeutung besitzen.

3. Verbindungen der Formel (I) nach Anspruch 2, worin:
p und r für 1 stehen;
die gestrichelte Bindung für eine Einfach- oder Doppelbindung steht;
R_{1a,b,c,d} für Wasserstoff stehen oder eine der Gruppen R_{1a,b,c,d} für ein Halogen steht und die anderen Gruppen R_{1a,b,c,d} für Wasserstoff stehen;
R_{2a,b} für Wasserstoff stehen oder eine der Gruppen R_{2a,b} für eine (C₁-C₅)-Alkylgruppe, vorzugsweise Methyl, steht, und die andere Gruppe für Wasserstoff steht;
R₃ für Wasserstoff steht;
R₄ in 4-Position unter den folgenden Arylen oder Heteroarylen ausgewählt ist:
- Pyridin
- Phenyl
- Pyrazol.

4. Verbindungen der Formel (I) nach Anspruch 2, worin X für das Kohlenstoffatom steht und die gestrichelte Bindung für eine Doppelbindung steht, R₄ für Phenyl oder Pyridin steht und R_{1a,b,c,d}, R_{2a,b}, R₃, R₅ bis R₇, p und r die in Anspruch 1 angegebene Bedeutung besitzen.

5. Verbindungen der Formel (I) nach Anspruch 2, worin X für das Kohlenstoffatom steht und die gestrichelte Bindung für eine Einfachbindung steht, R₄ für Phenyl oder Pyridin steht und R_{1a,b,c,d}, R_{2a,b}, R₅ bis R₇, p und r die in Anspruch 1 angegebene Bedeutung besitzen.

6. Verbindungen der Formel (I) nach Anspruch 2, worin X für das Kohlenstoffatom steht und die gestrichelte Bindung für eine Einfachbindung steht, R₄ für Phenyl, Pyridin oder Pyrazol steht und R_{1a,b,c,d}, R_{2a,b}, R₅ bis R₇, p und r die in Anspruch 1 angegebene Bedeutung besitzen.

7. Verfahren zur Herstellung einer Verbindung der Formel (I) nach einem der Ansprüche 1 bis 6, **dadurch gekennzeichnet, daß** man eine Verbindung der Formel (IV): 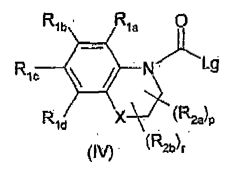 worin:
- R_{1a,b,c,d}, R_{2a,b}, p und r die in Anspruch 1 für die Verbindungen der Formel (I) angegebene Bedeutung besitzen und
- Lg für eine Abgangsgruppe steht;
mit einer Verbindung der Formel (V) 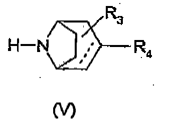 worin:
- R₃ und R₄ die in Anspruch 1 für die Verbindungen der Formel (I) angegebene Bedeutung besitzen;
umsetzt und
die so erhaltene Verbindung gegebenenfalls in eines ihrer Salze umwandelt.

8. Arzneimittel, **dadurch gekennzeichnet, daß** es eine Verbindung der Formel (I) nach einem der Ansprüche 1 bis 6 oder ein Additionssalz dieser Verbindung mit einer pharmazeutisch unbedenklichen Säure oder auch ein Hydrat oder ein Solvat der Verbindung der Formel (I) enthält.

9. Pharmazeutische Zusammensetzung, **dadurch gekennzeichnet, daß** sie eine Verbindung der Formel (I) nach einem der Ansprüche 1 bis 6 oder ein pharmazeutisch unbedenkliches Salz, ein Hydrat oder ein Solvat dieser Verbindung sowie mindestens einen pharmazeutisch unbedenklichen Hilfsstoff enthält.

10. Verwendung einer Verbindung nach einem der Ansprüche 1 bis 6 zur Herstellung eines Arzneimittels zur Behandlung von Obesitas, Diabetes, Insulinresistenz, metabolischem Syndrom, Cushing-Syndrom, Hypertonie, Atherosklerose, kognitiven Störungen und Demenz, Glaukomen, Osteoporose und pathologischen Zuständen, die die Aktivierung des Immunsystems erfordern.
